# EUROPÄISCHE PATENTSCHRIFT

(11) **EP 1 476 164 B1**
(45) Veröffentlichungstag und Bekanntmachung des Hinweises auf die Patenterteilung: **02.11.2005**
(21) Anmeldenummer: 03737277.8
(22) Anmeldetag: 27.01.2003
(51) Int. Cl.: A61K 31/498, C07D 405/12, C07D 401/12, C07D 409/12, C07D 403/12, A61P 9/00

(54) **CHINOXALINONE UND IHRE VERWENDUNG INSBESONDERE IN DER BEHANDLUNG VON CARDIOVASKULAREN ERKRAUNKUNGEN**
QUINOXALINONES AND THEIR USE ESPECIALLY IN THE TREATMENT OF CARDIOVASCULAR DISEASES
QUINOXALINONES ET LEUR UTILISATION NOTAMMENT POUR TRAITER DES AFFECTIONS CARDIOVASCULAIRES

(30) Priorität: 08.02.2002 DE 10205219
(43) Veröffentlichungstag der Anmeldung: 17.11.2004
(73) Patentinhaber: Bayer HealthCare AG, 51368 Leverkusen (DE)
(72) Erfinder: ERGÜDEN, Jens-Kerim, 42489 Wülfrath (DE); KOLKHOF, Peter, 42113 Wuppertal (DE); CASTRO-PALOMINO, Julio, 08330 Premia de Mar, Barcelona (ES); KUHL, Alexander, 58093 Hagen (DE); KAST, Raimund, 42349 Wuppertal (DE); STASCH, Johannes-Peter, 42651 Solingen (DE); TINEL, Hanna, 42111 Wuppertal (DE); MÜNTER, Klaus, 42489 Wülfrath (DE); LUSTIG, Klemens, 42113 Wuppertal (DE); PERNERSTORFER, Josef, 40721 Hilden (DE); BECHEM, Martin, 42111 Wuppertal (DE); HÜSER, Jörg, 42327 Wuppertal (DE)
(86) Internationale Anmeldenummer: PCT/EP2003/000782
(87) Internationale Veröffentlichungsnummer: WO 2003/066057

(56) Entgegenhaltungen:
- EP-A- 0 345 629
- EP-A- 0 556 738
- DE-A- 4 341 663

## Beschreibung

Die Erfindung betrifft Chinoxalinone, ein Verfahren zu ihrer Herstellung sowie ihre Verwendung zur Herstellung von Arzneimitteln zur Behandlung und/oder Prophylaxe von Krankheiten, insbesondere von kardiovaskulären Erkrankungen.

Acetylcholin ist der Überträgerstoff des parasympathischen Nervensystems. Dieser Teil des vegetativen Nervensystems hat entscheidenden Einfluss auf fundamentale Prozesse verschiedenster Organfunktionen, wie z.B. Lunge, Blase, Magen und Darm, Drüsen, Gehirn, Auge, Blutgefäße und Herz.

Acetylcholin selbst ist aufgrund der sehr schnellen Inaktivierung durch die Acetylcholinesterase therapeutisch nicht anwendbar, seine Wirkung kann aber durch direkte Parasympathomimetika, wie z.B. das Carbachol, imitiert werden. Wirkstoffe, die wie Acetylcholin an den muskarinischen (M) Acetylcholinrezeptoren agonistisch wirken, können somit, je nach Organ- oder Gewebesystem, zahlreiche Funktionen beeinflussen und steuern. Beispielsweise kann eine Aktivierung muskarinischer Acetylcholinrezeptoren im Gehirn das Gedächtnis und Prozesse von Lernvorgängen und Schmerzverarbeitung beeinflussen.

Durch Anwendung rezeptorsubtypspezifischer Agonisten ist man beispielsweise in der Lage, über den muscarinischen M2 Acetylcholinrezeptor, der besonders stark in Herzmuskelzellen exprimiert wird, die Herzfrequenz und die Kontraktilitaet nach beta-adrenerger Stimulation zu reduzieren (B. Rauch, F. Niroomand, *J. Eur. Heart.* **1991,** *12*, 76-82). Beide Effekte reduzieren den myocardialen Sauerstoffverbrauch.

EP 0 345 629 beispielsweise beschreibt Diazepinone dieser Aktivität. EP 0 556 738 offenbart Dihydro-oxo-pyridin derivate für die Behandlung von kardiovaskulären Erkrankungen.

Den erfindungsgemäßen Verbindungen strukturell ähnliche Substanzen sind in anderen Indikationen bzw. für andere Wirkmechanismen bekannt. So beschreiben beispielsweise WO 00/00478, EP-A 0 728 481 und EP-A 0 509 398 Chinoxalinon-Derivate zur Behandlung von HIV-Infektionen, DE 4341663 beschreibt Chinoxalinon-Derivate als Endothelinrezeptor-Antagonisten, WO 98/09987 beschreibt Chinoxalinon-Derivate als Thrombin- Inhibitoren, WO 94/11355 beschreibt 3,4-Dihydro-1-phenyl-2(1H)-chinoxalinon-Derivate zur Behandlung von kardiovaskulären Erkrankungen und US 3,654,275 beschreibt Chinoxalincarboxamide als antiinflammatorisch wirksame Verbindungen.

Aufgabe der vorliegenden Erfindung ist die Bereitstellung von Arzneimitteln für die Behandlung von Erkrankungen, insbesondere kardiovaskulären Erkrankungen.

Die vorliegende Erfindung betrifft Verbindungen der Formel 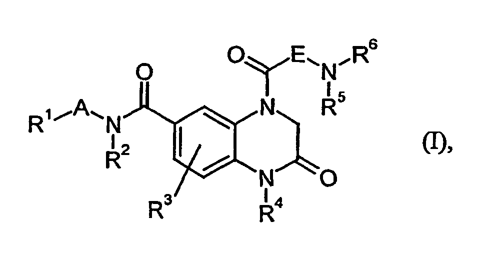 in welcher
- A: für eine C₁-C₆-Alkandiylkette steht, die gegebenenfalls mit einer oder zwei Hydroxygruppen substituiert ist,
- E: für eine C₁-C₆-Alkandiylkette steht,
- R¹: für Heteroaryl steht, wobei Heteroaryl gegebenenfalls substituiert ist mit 1 bis 3 Substituenten, unabhängig voneinander ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl,
- R²: für Wasserstoff, Alkyl oder Cycloalkyl steht,
- R³: für Wasserstoff, Halogen, Alkyl oder Alkoxy steht,
- R⁴: für Alkyl oder Cycloalkyl steht, wobei Alkyl und Cycloalkyl gegebenenfalls substituiert sind mit 1 bis 3 Substituenten, unabhängig voneinander ausgewählt aus der Gruppe bestehend aus Halogen, Hydroxy, Amino, Alkyl, Alkoxy, Alkylamino, Alkoxycarbonyl, Aminocarbonyl und Alkylaminocarbonyl,
- R⁵: für Wasserstoff, Alkyl oder Cycloalkyl steht,
und
- R⁶: für Alkyl, Cycloalkyl, Heterocyclyl, Aryl oder Heteroaryl steht, wobei Alkyl, Cycloalkyl, Heterocyclyl, Aryl oder Heteroaryl gegebenenfalls substituiert sind mit 1 bis 3 Substituenten unabhängig voneinander ausgewählt aus der Gruppe bestehend aus Halogen, Hydroxy, Amino, Trifluormethyl, Nitro, Cyano, Alkyl, das seinerseits mit einer oder zwei Hydroxygruppen substituiert sein kann, Alkoxy, Alkylamino, Alkoxycarbonyl, Aminocarbonyl und Alkylaminocarbonyl,
oder
- R⁵ und R⁶: bilden zusammen mit dem Stickstoffatom an das sie gebunden sind einen stickstoffhaltigen Heterocyclyl-Ring, wobei Heterocyclyl gegebenenfalls substituiert ist mit 1 bis 3 Substituenten unabhängig voneinander ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl.

Die erfindungsgemäßen Verbindungen können auch in Form ihrer Salze, Solvate oder Solvate der Salze vorliegen.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Dihydroabiethylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylaminocarbonyl und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N*-Diethylamino, *N*-Ethyl-*N*-methylamino, N-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, tert.-Butylaminocarbonyl, n-Pentylaminocarbonyl, n-Hexylaminocarbonyl, *N*,*N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-t-Butyl-*N*-methylaminocarbonyl, *N*-Ethyl-*N*-n-pentylamino-carbonyl und *N*-n-Hexyl-*N*-methylaminocarbonyl.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Alkandiyl steht für einen geradkettigen oder verzweigten gesättigten Alkandiylrest mit 1 bis 6 Kohlenstoffatomen. Bevorzugt ist ein geradkettiger oder verzweigter Alkandiylrest mit 1 bis 4 Kohlenstoffatomen. Beispielhaft und vorzugsweise seien genannt Methylen, Ethan-1,2-diyl, Ethan-1,1-diyl, Propan-1,3-diyl, Propan-1,2-diyl, Propan-2,2-diyl, Butan-1,4-diyl, Butan-1,3-diyl, Butan-2,4-diyl, Pentan-1,5-diyl, Pentan-2,4-diyl, 2-Methyl-pentan-2,4-diyl.

Cycloalkyl steht für eine Cycloalkylgruppe mit in der Regel 3 bis 8, bevorzugt 3 bis 6 Kohlenstoffatomen, beispielhaft und vorzugsweise für Cyclopropyl, Cyclobutyl, Cyclopentyl, Cyclohexyl und Cycloheptyl.

Aryl steht für einen mono- bi- oder tricyclischen aromatischen, carbocyclischen Rest mit in der Regel 6 bis 14 Kohlenstoffatomen, beispielhaft und vorzugsweise für Phenyl, Naphthyl und Phenanthrenyl.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

Heterocyclyl steht für einen mono- oder polycyclischen, vorzugsweise mono- oder bicyclischen, nicht-aromatischen heterocyclischen Rest mit in der Regel 4 bis 10, vorzugsweise 5 bis 8 Ringatomen und bis zu 3, vorzugsweise bis zu 2 Heteroatomen und/oder Heteroatomgruppen aus der Reihe N, O, S, SO, SO₂. Die HeterocyclylReste können gesättigt oder teilweise ungesättigt sein. Bevorzugt sind 5- bis 8-gliedrige, monocyclische gesättigte Heterocyclylreste mit bis zu zwei Heteroatomen aus der Reihe O, N und S, wie beispielhaft und vorzugsweise Tetrahydrofuran-2-yl, Pyrrolidin-2-yl, Pyrrolidin-3-yl, Pyrrolinyl, Piperidinyl, Morpholinyl, Perhydroazepinyl.

Halogen steht für Fluor, Chlor, Brom und Jod.

Ein Symbol * an einer Bindung bedeutet die Verknüpfungsstelle im Molekül.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt sind Verbindungen der Formel (I),
in welcher
- A: für eine C₁-C₆-Alkandiylkette steht,
- E: für eine C₁-C₆-Alkandiylkette steht,
- R¹: für Heteroaryl steht, wobei Heteroaryl gegebenenfalls substituiert ist mit 1 bis 3 Substituenten, unabhängig voneinander ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl,
- R²: für Wasserstoff steht,
- R³: für Wasserstoff steht,
- R⁴: für Alkyl oder Cycloalkyl steht, wobei Alkyl und Cycloalkyl gegebenenfalls substituiert sind mit 1 bis 3 Substituenten, unabhängig voneinander ausgewählt aus der Gruppe bestehend aus Alkyl, Alkoxy und Alkylamino,
- R⁵: für Wasserstoff steht,
und
- R⁶: für Alkyl, Cycloalkyl, Heterocyclyl, Aryl oder Heteroaryl steht, wobei Alkyl, Cycloalkyl, Heterocyclyl, Aryl oder Heteroaryl gegebenenfalls substituiert sind mit 1 bis 3 Substituenten unabhängig voneinander, ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl.

Besonders bevorzugt sind Verbindungen der Formel (I),
in welcher
- A: für Methylen oder Ethan-1,1-diyl steht,
- E: für Methylen steht,
- R¹: für 5- oder 6-gliedriges Heteroaryl steht, wobei Heteroaryl gegebenenfalls substituiert ist mit 1 bis 3 Substituenten, unabhängig voneinander ausgewählt aus der Gruppe bestehend aus Halogen, Hydroxy, Amino, Alkyl, Alkoxy, Alkylamino, Alkoxycarbonyl, Aminocarbonyl und Alkylaminocarbonyl,
- R²: für Wasserstoff steht,
- R³: für Wasserstoff steht,
- R⁴: für Cycloalkyl steht, wobei Cycloalkyl gegebenenfalls substituiert ist mit 1 oder 2 Alkylsubstituenten,
- R⁵: für Wasserstoff steht,
und
- R⁶: für Alkyl oder Cycloalkyl steht, wobei Cycloalkyl gegebenenfalls substituiert ist mit 1 bis 3 Substituenten, unabhängig voneinander ausgewählt aus der Gruppe bestehend aus Alkyl, Alkoxy, Alkylamino, Alkoxycarbonyl, Aminocarbonyl und Alkylaminocarbonyl.

Ganz besonders bevorzugt sind Verbindungen der Formel (I),
in welcher
- A: für Ethan-1,1-diyl steht,
- E: für Methylen steht,
- R¹: für 6-gliedriges Heteroaryl steht, wobei Heteroaryl gegebenenfalls substituiert ist mit 1 oder 2 Substituenten, unabhängig voneinander ausgewählt aus der Gruppe bestehend aus Methyl, Ethyl, Methoxy und Ethoxy,
- R²: für Wasserstoff steht,
- R³: für Wasserstoff steht,
- R⁴: für Cyclopropyl steht,
- R⁵: für Wasserstoff steht,
und
- R⁶: für Cyclohexyl oder Cyclopentyl steht, wobei Cyclohexyl oder Cyclopentyl gegebenenfalls substituiert sind mit 1 oder 2 Substituenten, unabhängig voneinander ausgewählt aus der Gruppe bestehend aus Methyl, Ethyl, Methoxy und Ethoxy.

Ebenfalls ganz besonders bevorzugt sind Verbindungen der Formel (I), in welcher A für Ethan-1,1-diyl steht.

Ebenfalls ganz besonders bevorzugt sind Verbindungen der Formel (I), in welcher R¹-A- für einen Rest der Formel 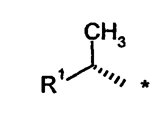 steht.

Ebenfalls ganz besonders bevorzugt sind Verbindungen der Formel (I), in welcher E für Methylen steht.

Ebenfalls ganz besonders bevorzugt sind Verbindungen der Formel (I), in welcher R¹ für 6-gliedriges Heteroaryl, insbesondere Pyridyl, steht.

Ebenfalls ganz besonders bevorzugt sind Verbindungen der Formel (I), in welcher R² für Wasserstoff steht.

Ebenfalls ganz besonders bevorzugt sind Verbindungen der Formel (I), in welcher R³ für Wasserstoff steht.

Ebenfalls ganz besonders bevorzugt sind Verbindungen der Formel (I), in welcher R⁴ für Cycloalkyl, insbesondere Cyclopropyl, steht.

Ebenfalls ganz besonders bevorzugt sind Verbindungen der Formel (I), in welcher R⁵ für Wasserstoff steht.

Ebenfalls ganz besonders bevorzugt sind Verbindungen der Formel (I), in welcher R⁶ für Cyclohexyl oder Cyclopentyl steht, wobei Cyclohexyl und Cyclopentyl gegebenenfalls substituiert sind mit 1 oder 2 Substituenten, unabhängig voneinander ausgewählt aus der Gruppe bestehend aus Methyl und Ethyl.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Die Erfindung betrifft weiterhin ein Verfahren zur Herstellung der Verbindungen der Formel (I), dadurch gekennzeichnet, dass Verbindungen der Formel 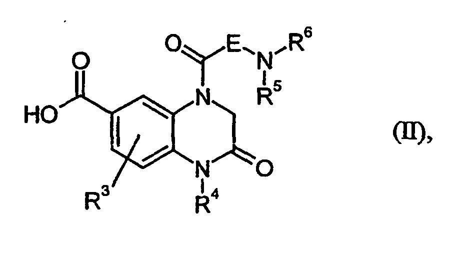 in welcher
- E, R³, R⁴, R⁵ und R⁶: die oben angegebene Bedeutung aufweisen,
mit Verbindungen der Formel 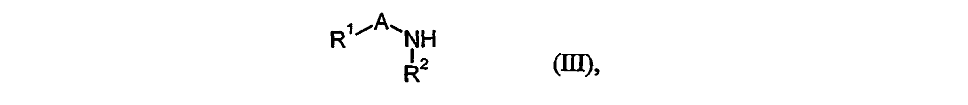 in welcher
- A, R¹ und R²: die oben angegebene Bedeutung aufweisen,
in Gegenwart von üblichen Kondensationsmitteln, gegebenenfalls in Gegenwart einer Base umgesetzt werden.

Die Umsetzung erfolgt gegebenenfalls in inerten Lösungsmitteln, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin, bevorzugt sind Tetrahydrofuran, Dimethylformamid oder Methylenchlorid.

Übliche Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin oder Diisopropylethylamin.

Bevorzugt ist die Kombination von N-(3-Dimethylaminoisopropyl)-N'-ethylcarbodiimid-Hydrochlorid (EDC), 1-Hydroxybenztriazol (HOBt) und Diisopropylethylamin in Methylenchlorid.

Verbindungen der Formel (III) sind bekannt oder können analog bekannten Verfahren hergestellt werden.

Zur Herstellung der Verbindungen der Formel (II) setzt man Verbindungen der Formel 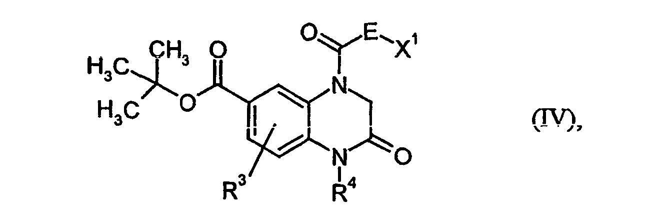 in welcher
- E, R³ und R⁴: die oben angegebene Bedeutung aufweisen und
- X¹: für Halogen, bevorzugt Brom oder Chlor, steht,
zunächst mit Verbindungen der Formel 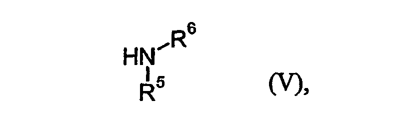 in welcher
- R⁵ und R⁶: die oben angegebene Bedeutung aufweisen
und anschließend mit Trifluoressigsäure zur Spaltung des tert.-Butylesters um.

Die Umsetzung der ersten Stufe erfolgt gegebenenfalls in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, 2-Butanon, Dimethylsulfoxid, Acetonitril oder Pyridin, bevorzugt sind Tetrahydrofuran oder Methylenchlorid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt Diisopropylethylamin oder Triethylamin.

Die Umsetzung der zweiten Stufe erfolgt in inerten Lösungsmitteln, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, oder andere Lösungsmittel Dimethylformamid oder Tetrahydrofuran, bevorzugt ist Methylenchlorid.

Verbindungen der Formel (V) sind bekannt oder können analog bekannten Verfahren hergestellt werden.

Zur Herstellung der Verbindungen der Formel 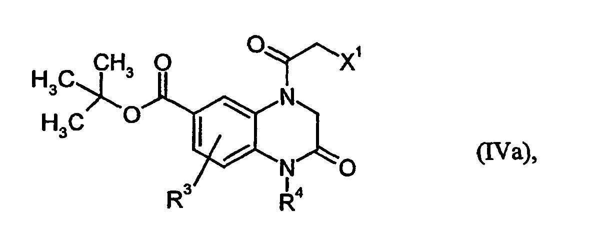 wobei es sich um Verbindungen der Formel (IV) handelt, in welcher
- E: für Methylen steht und
- R³, R⁶ und X¹: die oben angegebene Bedeutung aufweisen,
setzt man Verbindungen der Formel 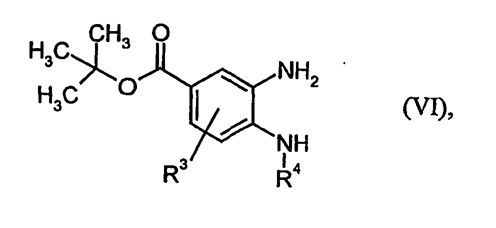 in welcher
- R³ und R⁴: die oben angegebene Bedeutung aufweisen,
mit Verbindungen der Formel 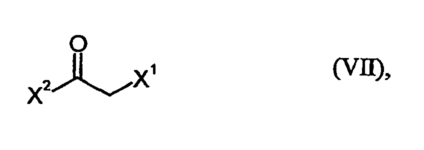 in welcher
- X¹: die oben angegebene Bedeutung aufweist, und
- X²: für Halogen, bevorzugt Brom oder Chlor, steht,
um.

Die Reaktion erfolgt in zwei Stufen. Die Umsetzung der ersten Stufe erfolgt in inerten Lösungsmitteln mit 2 Äquivalenten der Verbindungen der Formel (VII) bezogen auf die Verbindungen der Formel (VI), in Gegenwart von 2 Äquivalenten einer Base, bevorzugt in einem Temperaturbereich von 0°C bis 50°C bei Normaldruck. Die zweite Stufe schließt sich ohne Aufarbeitung der Reaktionsmischung an und erfolgt durch Zugabe einer weiteren Base.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, 2-Butanon, Dimethylsulfoxid, Acetonitril oder Pyridin, bevorzugt ist Methylenchlorid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt für die erste Stufe ist Diisopropylethylamin oder Triethylamin, bevorzugt für die zweite Stufe ist DBU.

Verbindungen der Formel (VII) sind bekannt oder können analog bekannten Verfahren hergestellt werden.

Zur Herstellung der Verbindungen der Formel (VI), setzt man Verbindungen der Formel 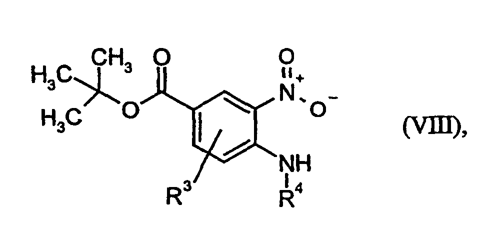 in welcher
- R³ und R⁴: die oben angegebene Bedeutung aufweisen,
mit Reduktionsmitteln in Gegenwart eines Katalysators um.

Die Umsetzung erfolgt in inerten Lösungsmitteln, bevorzugt in einem Temperaturbereich von Raumtemperatur bis 50°C bei Normaldruck.

Inerte Lösungsmittel sind beispielsweise Alkohole wie Methanol, Ethanol, Propanol, Isopropanol oder Butanol oder Ethylacetat oder Diethylether, bevorzugt sind Methanol oder Ethanol.

Reduktionsmittel ist beispielsweise Wasserstoff; Katalysatoren sind beispielsweise Zinndichlorid, Titantrichlorid oder Palladium auf Aktivkohle. Bevorzugt ist die Kombination Palladium auf Aktivkohle und Wasserstoff.

Zur Herstellung der Verbindungen der Formel (VIII), setzt man Verbindungen der Formel 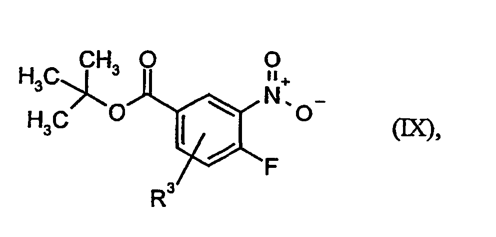 in welcher
- R³: die oben angegebene Bedeutung aufweist,
mit Verbindungen der Formel

R⁴―NH₂ (X),

in welcher
- R⁴: die oben angegebene Bedeutung aufweist,
gegebenenfalls in Gegenwart einer Base um.

Die Umsetzung erfolgt in inerten Lösungsmitteln, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, 2-Butanon, Dimethylsulfoxid, Acetonitril oder Pyridin, bevorzugt sind Tetrahydrofuran oder Methylenchlorid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt ist Triethylamin oder Diisopropylethylamin.

Verbindungen der Formel (IX) und (X) sind bekannt oder können analog bekannten Verfahren hergestellt werden.

In einer alternativen Methode zur Herstellung der Verbindungen der Formel (IV), setzt man Verbindungen der Formel 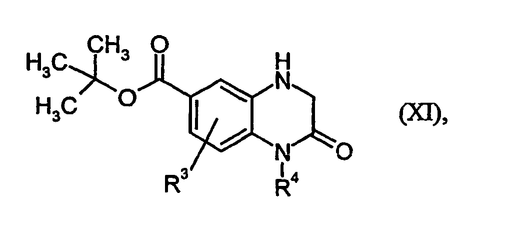 in welcher
- R³ und R⁴: die oben angegebene Bedeutung aufweisen,
mit Verbindungen der Formel 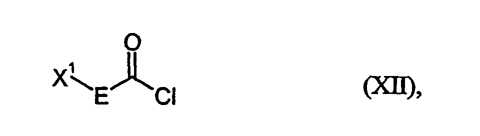 in welcher
- X¹ und E: die oben angegebene Bedeutung aufweisen,
gegebenenfalls in Gegenwart einer Base um.

Die Umsetzung erfolgt in inerten Lösungsmitteln, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, 2-Butanon, Dimethylsulfoxid, Acetonitril oder Pyridin, bevorzugt sind Tetrahydrofuran oder Methylenchlorid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt ist Triethylamin oder Diisopropylethylamin.

Verbindungen der Formel (XI) sind bekannt oder können analog bekannten Verfahren hergestellt werden (beispielsweise gemäß EP-A 0 509 398).

Verbindungen der Formel (XII) sind bekannt oder können analog bekannten Verfahren hergestellt werden.

Die Herstellung der erfindungsgemäßen Verbindungen kann durch das folgende Syntheseschema verdeutlicht werden.

### Syntheseschema:

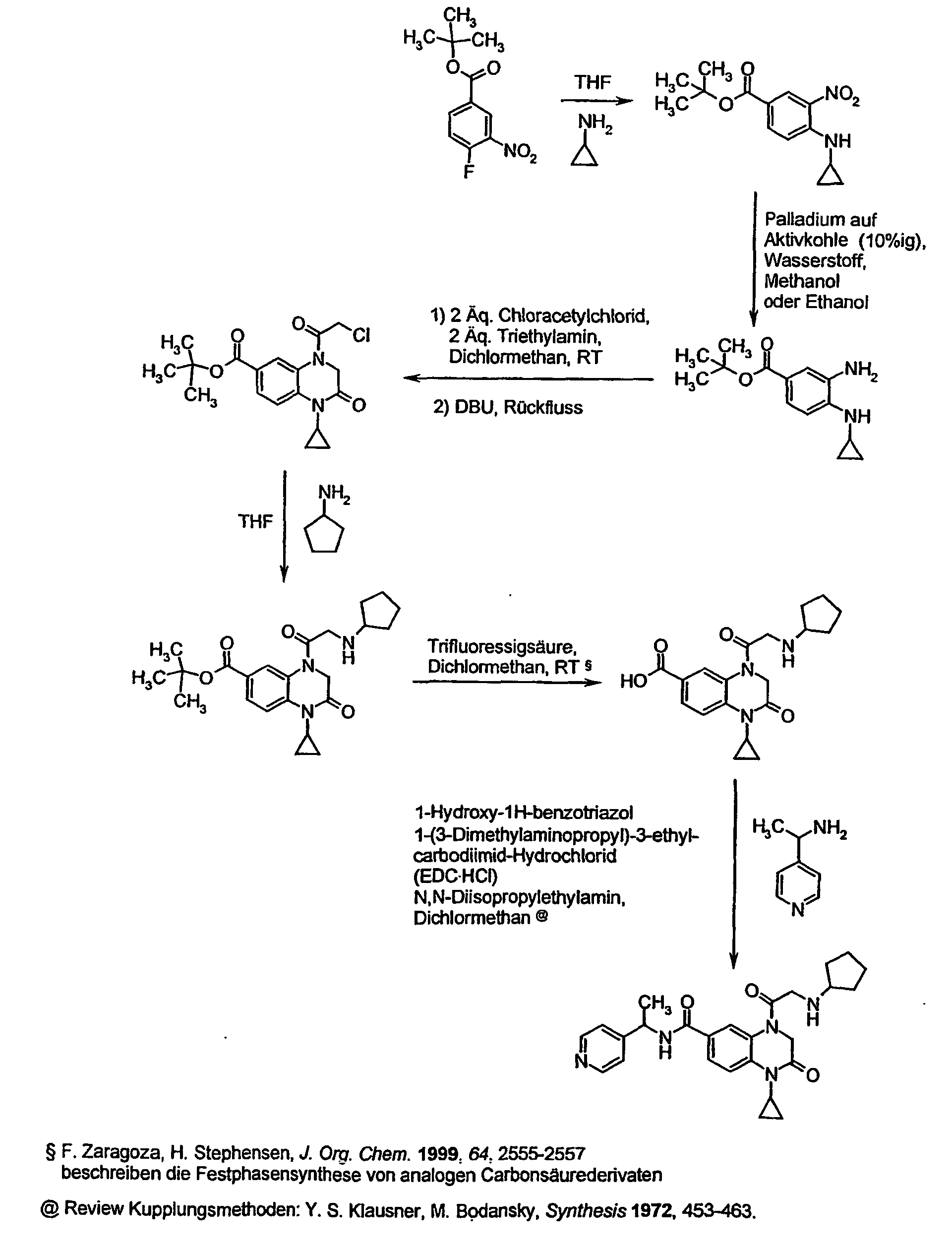

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Sie zeichnen sich als Agonisten des muskarinischen M2 Acetycholinrezeptors aus.

Die erfindungsgemäßen Verbindungen können aufgrund ihrer pharmakologischen Eigenschaften allein oder in Kombination mit anderen Wirkstoffen zur Behandlung und/oder Prophylaxe von kardiovaskulären Krankheiten, insbesondere von koronarer Herzkrankheit, Angina pectoris, Myocardinfarkt, Schlaganfall, Ateriosklerose, essentielle, pulmonale und maligne Hypertonie, Herzinsuffizienz, Herzversagen, kardiale Arrythmien oder Thromboembolischen Erkrankungen eingesetzt werden.

Weiterhin eignen sie sich zur Behandlung und/oder Prophylaxe von Erkrankungen am Auge (Glaukom), Magen und Darm (Atonien), des Gehirns (z.B. Morbus Parkinson, Morbus Alzheimer, chronisches Schmerzempfinden), Nierenversagen oder erektile oder renale Dysfunktionen.

Weiterer Gegenstand der vorliegenden Erfindung sind Arzneimittel, die mindestens eine erfindungsgemäße Verbindung, vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nicht überzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augen-präparationen, Vaginalkapseln, wässrige Suspensionen (Lotionen, Schüttelmixturen), lipophile Suspensionen, Salben, Cremes, Milch, Pasten, Streupuder oder Implantate, beispielsweise Stents.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 0,0001 bis 10 mg/kg, vorzugsweise etwa 0,001 bis 1 mg/kg Körpergewicht zur Erzielung wirksamer Ergebnisse zu verabreichen. Bei oraler Applikation beträgt die Menge etwa 0,1 bis 10 mg/kg, vorzugsweise etwa 0,5 bis 5 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- aq.: wässrig
- Boc: tert.-Butoxycarbonyl
- CDCl₃: Deuterochloroform
- DMSO: Dimethylsulfoxid
- DMF: Dimethylformamid
- d. Th.: der Theorie
- EDC: *N'*-(3-Dimethylaminopropyl)-*N*-ethylcarbodiimid x HCl
- eq.: Äquivalent
- ESI: Elektrospray-Ionisation (bei MS)
- Fp.: Schmelzpunkt
- ges.: gesättigt
- HATU: *O*-(7-Azabenzotriazol-1-yl)-*N,N,N',N'*-tetramethyluronium-Hexafluorphosphat
- HBTU: *O*-(Benzotriazol-1-yl)-*N*,*N*,*N'*,*N'*-tetramethyluronium-Hexafluorphosphat
- HOBt: 1-Hydroxy-1H-benzotriazol x H₂O
- h: Stunde
- HPLC: Hochdruck-, Hochleistungsflüssigchromatographie
- LC-MS: Flüssigchromatographie-gekoppelte Massenspektroskopie
- MS: Massenspektroskopie
- MeOH: Methanol
- NMR: Kernresonanzspektroskopie
- Pd/C: Palladium/Kohle
- proz.: Prozent
- R_{f}: Retentionsindex (bei DC)
- RT: Raumtemperatur
- Rₜ: Retentionszeit (bei HPLC)

### HPLC und LC-MS-Methoden:

### Methode 1:

Instrument: HP 1100 mit DAD-Detektion; Säule: Kromasil RP-18, 60mm x 2mm, 3.5µm; Eluent: A=5ml HClO₄/l H₂O, B=ACN; Gradient: 0 min 2%B, 0.5 min 2%B, 4.5 min 90%B, 6.5 min 90%B; Fluss: 0.75 ml/min, Temp.:30 Grad C, Detektion UV 210 nm.

### Methode 2:

Instrument: Micromass Platform LCZ, HP1100; Säule: Symmetry C18, 50 mm x 2.1 mm, 3.5 µm; Eluent B: Wasser + 0.1% Ameisensäure, Eluent A: Acetonitril + 0.1% Ameisensäure; Gradient: 0.0min 10%A → 4.0min 90%A → 6.0min 90%A; Ofen: 40°C, Fluss: 0.5ml/min, UV-Detektion: 208-400 nm.

### Methode 3:

Instrument: Finnigan MAT 900S, TSP: P4000,AS3000,UV30000HR; Säule: Symmetry C 18, 150 mm x 2.1 mm, 5.0 µm; Eluent C: Wasser, Eluent B: Wasser + 0.6g 35%ige HCl, Eluent A: Acetonitril; Gradient: 0.0min 2%A, 49%B, 49%C → 2.5min 95%A, 2.5%B, 2.5% C → 5.5min 2%A, 49%B, 49%C; Ofen: 70°C, Fluss: 1.2ml/min, UV-Detektion: 210 nm.

### Methode 4:

Instrument: Mieromass Quattro LCZ, HP1100; Säule: Symmetry C18, 50 mm x 2.1 mm, 3.5 µm; Eluent A: Acetonitril + 0.1 % Ameisensäure, Eluent B: Wasser + 0.1 % Ameisensäure; Gradient: 0.0min 10%A → 4.0min 90%A → 6.0min 90%A; Ofen: 40°C, Fluss: 0.5ml/min, UV-Detektion: 208-400 nm.

### Methode 5:

Präp. HPLC Enantiomerentrennung; Packungsmaterial: Daicel Chiralpak AD 250*20 mm ID: ADOOCJ-GL001; Einsatz in g: 0.13; Eluent A: iso-Hexan, Eluent B: Ethanol + 0.2 % DEA; Gradient: 0.0min 30%A → 21min 30%A; Injektionsvolumen: 1000 µl Temperatur: 25°C, Fluss: 10 ml/min, Wellenlänge: 250 nm, Range: 1.

### Methode 6:

Instrument: Micromass Platform LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0min 90%A → 4.0min 10%A → 6.0min 10%A; Ofen: 40°C, Fluss: 0.5ml/min, UV-Detektion: 208-400 nm.

### Methode 7:

Instrument: Micromass Quattro LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0min 90%A → 4.0min 10%A → 6.0min 10%A; Ofen: 40°C, Fluss: 0.5ml/min, UV-Detektion: 208-400 nm.

### Methode 8:

Instrument: MS: Micromass ZQ; HPLC: Waters Alliance 2790; Säule: Symmetry C 18, 50 mm x 2.1 mm, 3.5 µm; Eluent B: Acetonitril + 0.05% Ameisensäure, Eluent A: Wasser + 0.05% Ameisensäure; Gradient: 0.0min 10%B → 3.5min 90%B → 5.5min 90%B; Ofen: 50 °C, Fluss: 0.8ml/min, UV-Detektion: 210 nm

### Ausgangsverbindungen:

### Beispiel I

### 4-Fluor-3-nitrobenzoesäure-tert-butylester

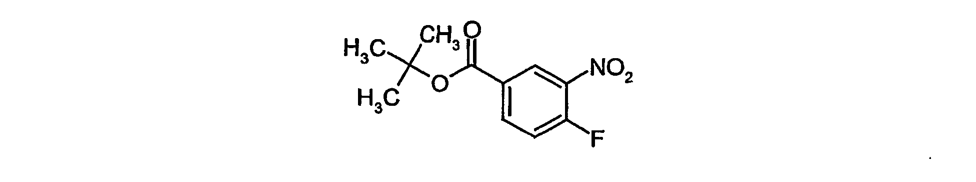

21.5 g (116.15 mmol) 4-Fluor-3-nitrobenzoesäure und 30.5 g (139.4 mmol) t-Butyltrichloracetimidat werden unter Argonatmosphäre in 250 ml Diethylether vorgelegt. Es werden 0.64 g (4.52 mmol) Bortrifluorid-Diethylether-Komplex zugetropft und der Ansatz 16 Stunden bei Raumtemperatur nachgerührt.

Man gibt 6 g festes Natriumhydrogencarbonat zu dem Reaktionsgemisch und engt im Vakuum ein. Der erhaltene Rückstand wird über Kieselgel chromatographisch gereinigt (Laufmittelgradient Cyclohexan → Cyclohexan-Ethylacetat 1:1).

Man erhält 17.8 g (64% d. Th.) Produkt.
¹H-NMR (300MHz, DMSO-d₆): δ = 1.57 (s, 9H), 7.2 (dd, 1H), 8.25-8.3 (m, 1H), 8.52 (dd, 1H)
MS (ESIpos): m/z = 242 (M+H)⁺
HPLC (Methode 1): Rₜ = 5.07 min

### Beispiel II

### 4-(Cyclopropylamin)-3-nitrobenzoesäure-tert-butylester

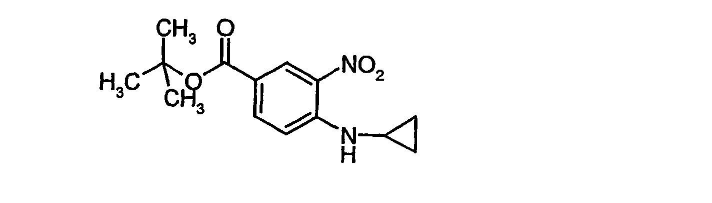

7.8 g (32.3 mmol) der Verbindung aus Beispiel I werden in 150 ml Tetrahydrofuran vorgelegt. Bei 0°C wird eine Lösung aus 3.88 g (67.9 mmol) Cyclopropylamin in 50 ml Tetrahydrofuran zugegeben. Man rührt 30 Minuten bei 0°C, dann 16 Stunden bei Raumtemperatur.

Das Reaktionsgemisch wird im Vakuum eingeengt. Man nimmt den Rückstand in 500 ml Ethylacetat auf und wäscht dreimal mit 100 ml Wasser und einmal mit 100 ml gesättigter Natriumchloridlösung. Es wird über Natriumsulfat getrocknet und im Vakuum eingeengt.

Man erhält 8.95 g (99% d. Th.) Produkt.
¹H-NMR (300MHz, DMSO-d₆): δ = 0.64-0.69 (m, 2H), 0.87-0.93 (m, 2H), 1.54 (s, 9H), 2.67-2.71 (m, 1H), 7.44 (d, 1H), 8.01 (dd, 1H), 8.33 (br.s, 1H), 8.54 (d, 1H)
MS (DCI): m/z = 296 (M+NH₄)⁺
HPLC (Methode 1): Rₜ = 5.47 min

### Alternativsynthese zu Beispiel II:

1 g (3.88 mmol) der Verbindung aus Beispiel XXXIII werden in Tetrahydrofuran (15 ml) vorgelegt. 0.44 g (7.76 mmol) Cyclopropylamin werden bei Raumtemperatur zugegeben. Die Lösung wird 2 Stunden bei 55°C gerührt. Der Ansatz wird dann auf Eiswasser (50 ml) gegossen. Der ausfallende Feststoff wird abgesaugt und getrocknet. Man erhält 0.65 g (58% d. Th.) Produkt.

Die in Tabelle 1 aufgeführten Verbindungen werden analog der Verbindung aus Beispiel II hergestellt.

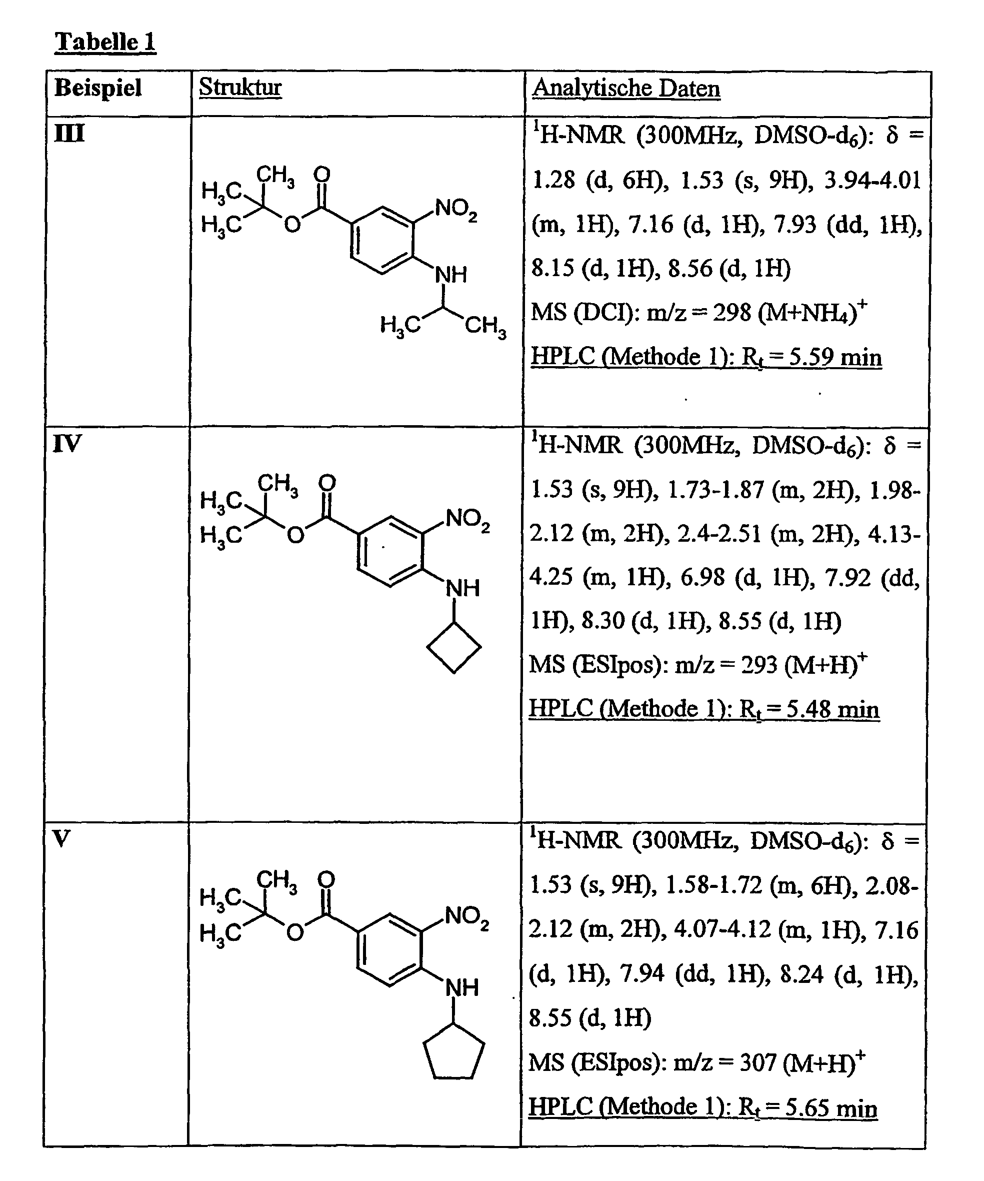

### Beispiel VI

### 3-Amino-4-(cyclopropylamin)benzoesäure-tert-butylester

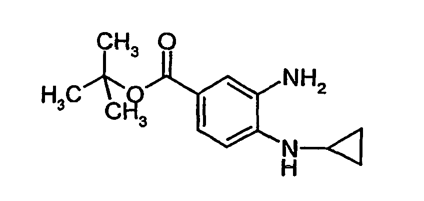

8.85 g (31.8 mmol) der Verbindung aus Beispiel II werden unter Argonatmosphäre in 400 ml Methanol vorgelegt und mit 0.30 g (1.33 mmol) Palladium auf Aktivkohle (10% Pd) versetzt. Man rührt unter Wasserstoffatmosphäre bei Normaldruck über Nacht. Der Ansatz wird über Celite filtriert und das Filtrat im Vakuum eingeengt. Man trocknet 2 Stunden im Hochvakuum und setzt das erhaltene Produkt (8.20 g, 94% d. Th.) ohne Verzögerung weiter um.
¹H-NMR (200MHz, DMSO-d₆): δ = 0.37-0.47 (m, 2H), 0.68-0.80 (m, 2H), 1.48 (s, 9H), 2.35-2.45 (m, 1H), 4.67 (br. s, 2H), 5.64 (br. s, 1H), 6.75 (d, 1H), 7.08 (d, 1H), 7.15 (dd, 1H)
MS (ESIpos): m/z = 249 (M+H)⁺
HPLC (Methode 1): Rₜ = 4.18 min

Die in Tabelle 2 aufgeführten Verbindungen werden analog der Verbindung aus Beispiel IV hergestellt. Als Lösungsmittel wird Ethanol verwendet. Die erhaltenen Produkte werden ohne Verzögerung weiter umgesetzt.

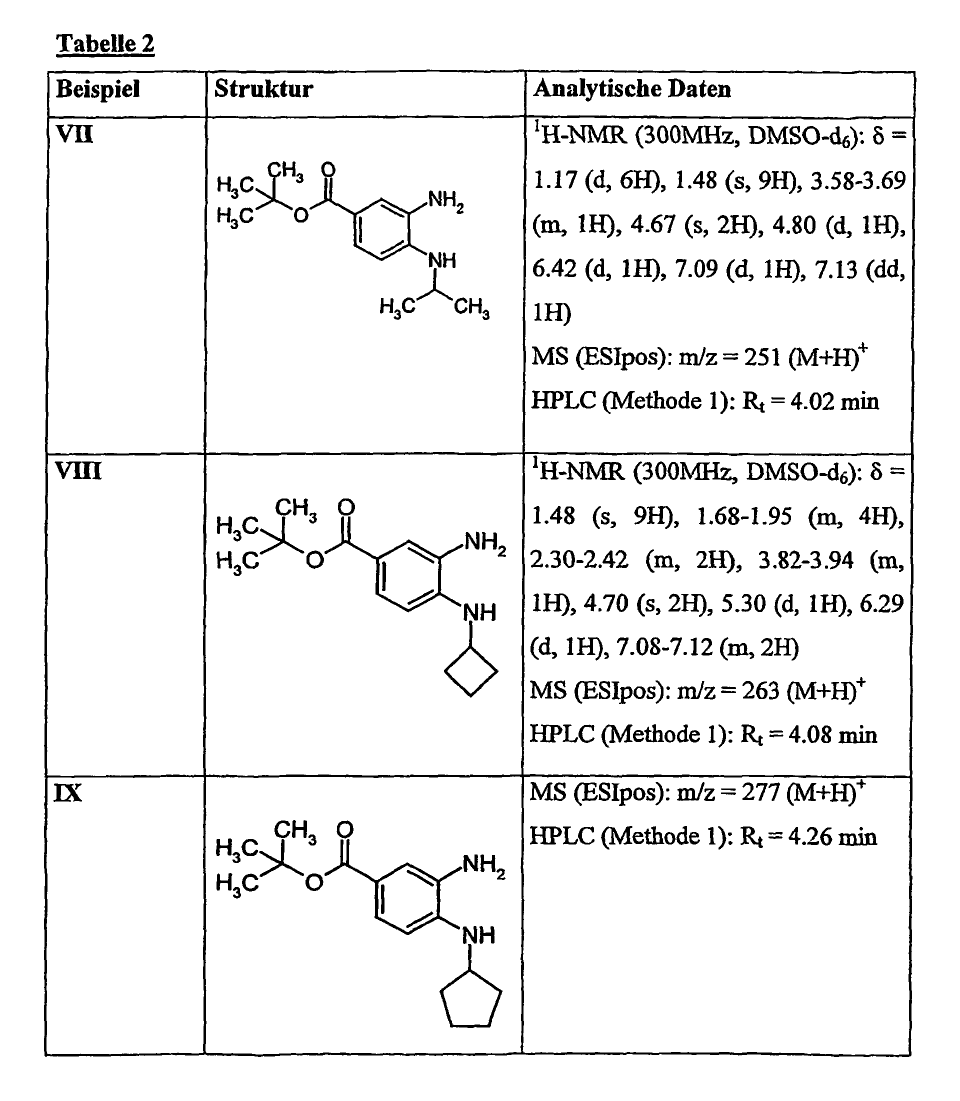

### Beispiel X

### 4-(Chloracetyl)-1-cyclopropyl-2-oxo-1,2,3,4-tetrahydro-6-chinoxalincarbonsäure-tert-butylester

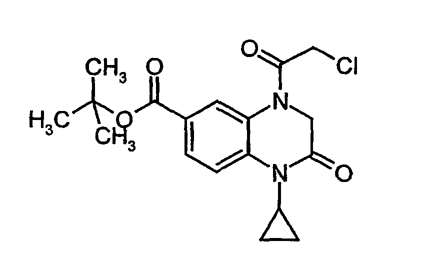

7.90 g (31.8 mmol) der Verbindung aus Beispiel VI werden in 200 ml Dichlormethan vorgelegt und bei 0°C mit 8.98 g (79.5 mmol) Chloressigsäurechlorid versetzt. Man rührt 30 Minuten bei Raumtemperatur nach. Bei 0°C werden 11.2 ml (79.5 mmol) Triethylamin zugegeben. Man rührt 4 Stunden bei Raumtemperatur. Anschließend werden 7.12 ml (7.26 g, 47.7 mmol) 1,8-Diaza-bicyclo(5.4.0)undec-7-en zugegeben und der Ansatz auf Rückfluss erhitzt. Nach 16 Stunden wird das Reaktionsgemisch abgekühlt und im Vakuum eingeengt.

Der Rückstand wird über Kieselgel chromatographisch gereinigt (Laufmittelgradient Cyclohexan → Cyclohexan-Ethylacetat 1:1).

Man erhält 4.69 g (62% d. Th.) Produkt als amorphen Feststoff.
¹H-NMR (400MHz, CDCl₃): δ = 0.68-0.72 (m, 2H), 1.16-1.21 (m, 2H), 1.60 (s, 9H), 2.78-2.82 (m, 1H), 4.21 (s, 2H), 4.51 (br. s, 2H), 7.46 (d, 1H), 7.98 (m, 2H)
MS (DCI): m/z = 382 (M+NH₄)⁺
HPLC (Methode 1): Rₜ = 4.72 min

### Beispiel XI

### 4-(Chloracetyl)-1-isopropyl-2-oxo-1,2,3,4-tetrahydro-6-chinoxalincarbonsäure-tert-butylester

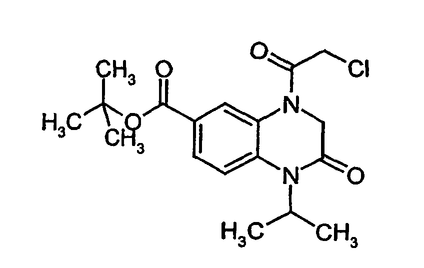

Die Darstellung erfolgt analog Beispiel X aus den entsprechenden Edukten.

Zur Aufarbeitung wird der Ansatz dreimal mit 100 ml Wasser und einmal mit 100 ml gesättiger Natriumchloridlösung gewaschen. Die organische Phase wird über Natriumsulfat getrocknet und im Vakuum eingeengt.

Der Rückstand wird über Kieselgel chromatographisch gereinigt (Laufinittelgradient Cyclohexan → Cyclohexan-Ethylacetat 2:1).
¹H-NMR (300MHz, DMSO-d₆): δ =1.45 (d, 6H), 1.54 (s, 9H), 4.37 (s, 2H), 4.51 (br. s, 2H), 4.61 (quintett, 1H), 7.49 (d, 1H), 7.81 (dd, 1H), 8.12 (d, 1H)
MS (DCI): m/z = 384 (M+NH₄)⁺
HPLC (Methode 1): Rₜ = 4.71 min

### Beispiel XII

### 4-(Chloracetyl)-1-cyclopentyl-2-oxo-1,2,3,4-tetrahydro-6-chinoxalincarbonsäure-tert-butylester

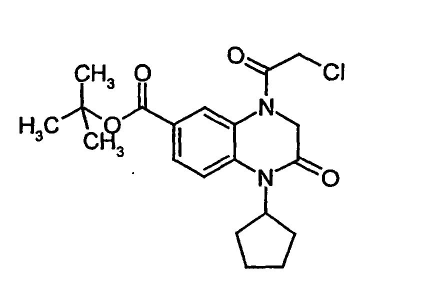

Die Darstellung erfolgt analog Beispiel X aus den entsprechenden Edukten.

Zur Aufarbeitung wird der Ansatz dreimal mit 100 ml Wasser und einmal mit 100 ml gesättiger Natriumchloridlösung gewaschen. Die organische Phase wird über Natriumsulfat getrocknet und im Vakuum eingeengt.

Man erhält 5.63 g (61% d. Th.) eines dunkelbraunen Schaumes. Das Produkt wird ohne weitere Reinigung weiter umgesetzt.
DC: R_{f}= 0.24 (Cyclohexan-Ethylacetat 5:1)

### Beispiel XIII

### 4-(Chloracetyl)-1-cyclobutyl-2-oxo-1,2,3,4-tetrahydro-6-chinoxalincarbonsäure-tert-butylester

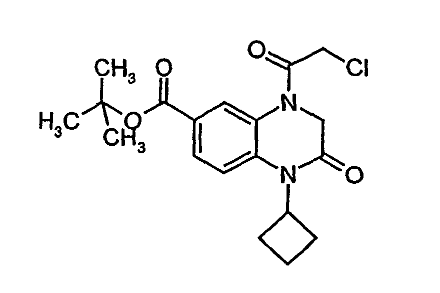

Die Darstellung erfolgt analog Beispiel X aus den entsprechenden Edukten.

Zur Aufarbeitung wird der Ansatz dreimal mit 100 ml Wasser und einmal mit 100 ml gesättiger Natriumchloridlösung gewaschen. Die organische Phase wird über Natriumsulfat getrocknet und im Vakuum eingeengt.

Man erhält 5.42 g (82% d. Th.) dunkelbraunen Schaum. Das Produkt wird ohne weitere Reinigung weiter umgesetzt.
R_{f}= 0.53 (Cyclohexan-Ethylacetat 2:1)

### Beispiel XIV

### 4-(N-Cyclopentylglycyl)-1-cyclopropyl-2-oxo-1,2,3,4-tetrahydro-6-chinoxalincarbonsäure-tert-butylester

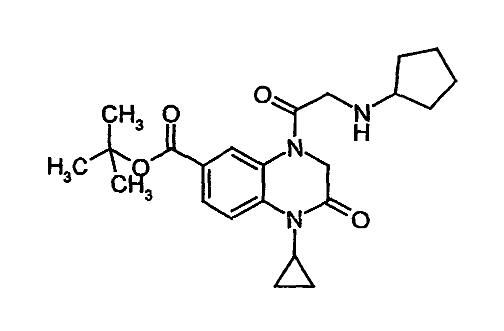

0.10 g (0.27 mmol) der Verbindung aus Beispiel X und 0.07 g (0.82 mmol) Cyclopentylamin werden in 10 ml Tetrahydrofuran gelöst und der Ansatz 6 Stunden bei 40°C gerührt. Man lässt 12 Stunden bei Raumtemperatur stehen.

Das Reaktionsgemisch wird im Vakuum eingeengt. Der Rückstand wird in 20 ml Ethylacetat aufgenommen und zweimal mit 10 ml gesättigter Natriumchloridlösung gewaschen. Die organische Phase wird über Natriumsulfat getrocknet und im Vakuum eingeengt. Das erhaltene Produkt wird ohne weitere Reinigung direkt weiter umgesetzt.
LCMS (Methode 2): Rₜ = 2.85 min
MS (ESIpos): m/z = 414 (M+H)⁺

Die in Tabelle 3 aufgeführten Verbindungen werden analog der Verbindung aus Beispiel XIV aus den entsprechenden Edukten hergestellt und ohne Reinigung weiter umgesetzt.

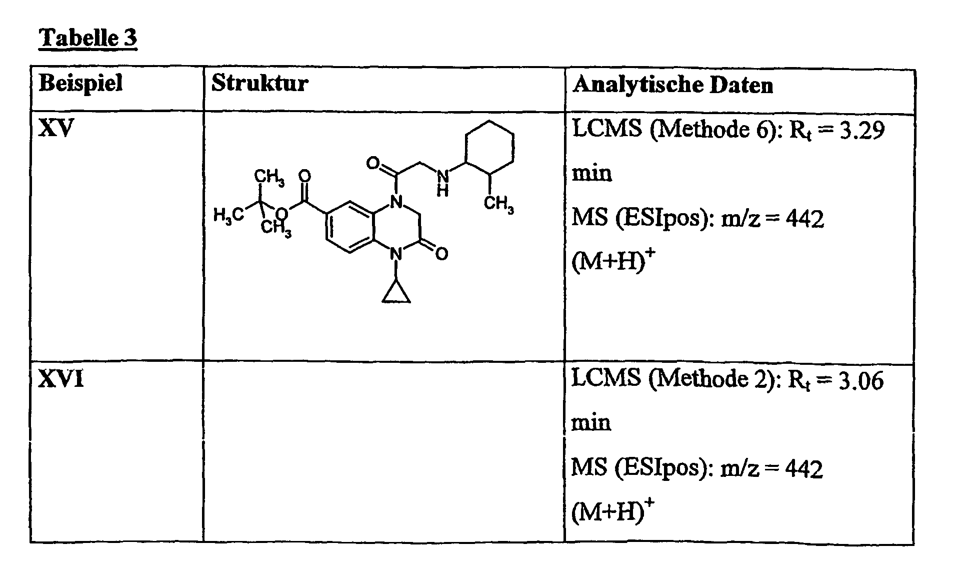

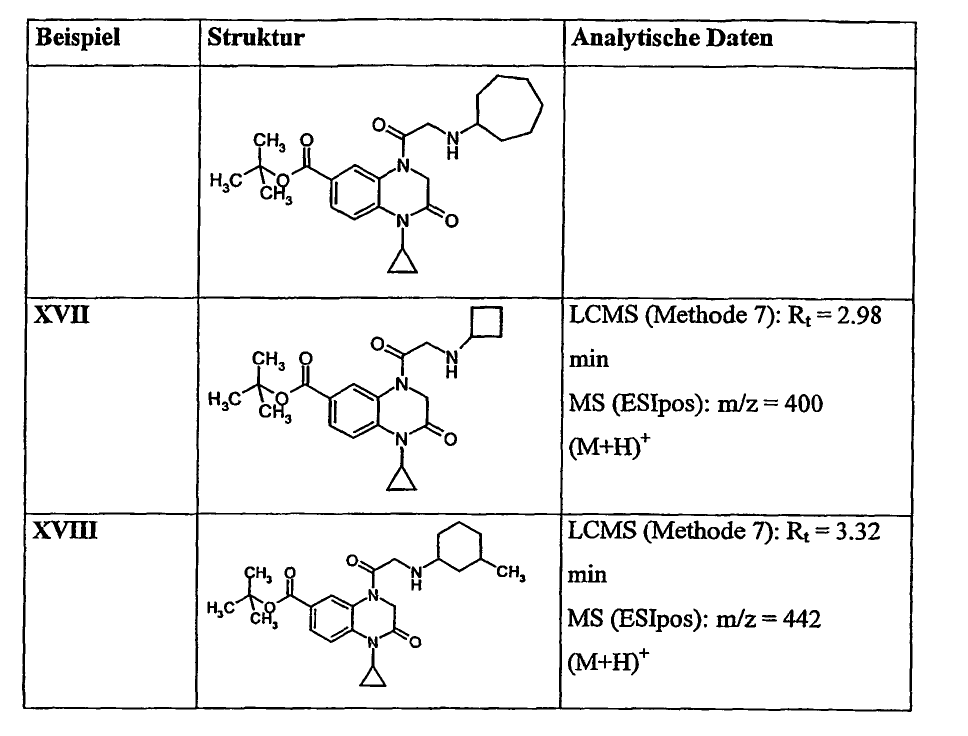

### Beispiel XIX

### 4-(N-Cyclohexylglycyl)-1-cyclopropyl-2-oxo-1,2,3,4-tetrahydro-6-chinoxalincarbonsäure-tert-butylester

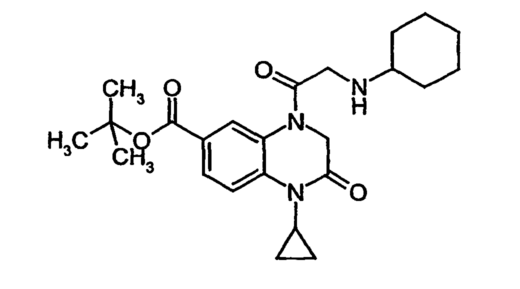

4.5 g (12.33 mmol) der Verbindung aus Beispiel X und 4.24 ml (3.67 g, 37.0 mmol) Cyclohexylamin werden in 175 ml Tetrahydrofuran gelöst und der Ansatz 72 h bei Raumtemperatur stehen gelassen.

Das Reaktionsgemisch wird im Vakuum eingeengt. Der Rückstand wird in 400 ml Ethylacetat aufgenommen und mit Wasser (3 x 100 ml) und einmal mit gesättigter Natriumchloridlösung (100 ml) gewaschen. Man trocknet über Natriumsulfat und engt im Vakuum ein.

Es wird über Kieselgel chromatographisch gereinigt [Laufmittelgradient Dichlormethan → Dichlormethan-Methanol 7.5 % (v/v)].

Man erhält 5.30 g (92% d. Th.) Produkt.
¹H-NMR (300MHz, DMSO-d₆): δ = 0.50-0.57 (m, 2H), 0.85-1.90 (m, 12H), 1.54 (s, 9H), 2.22-2.37 (m, 1H), 2.78-2.85 (m, 1H), 3.49 (br. s, 2H), 4.42 (s, 2H), 7.52 (d, 1H), 7.83 (dd, 1H), 8.06 (s, 1H)
MS (ESIpos): m/z = 428 (M+H)⁺
HPLC (Methode 1): Rₜ = 4.42 min

### Beispiel XX

### 4-(N-Cyclohexylglycyl)-1-isopropyl-2-oxo-1,2,3,4-tetrahydro-6-chinoxalin-carbonsäure-tert-butylester

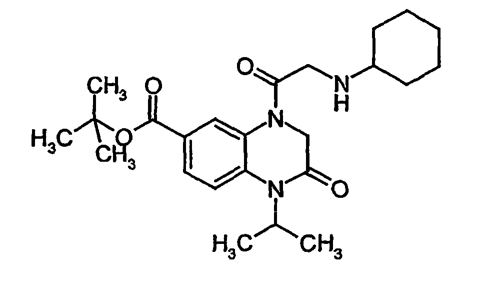

300 mg (0.82 mmol) der Verbindung aus Beispiel XI und 243 mg (2.45 mmol) Cyclohexylamin werden in 10 ml Tetrahydrofuran 16 Stunden bei Raumtemperatur gerührt.

Zur Aufarbeitung wird das Reaktionsgemisch im Vakuum eingeengt. Der Rückstand wird über Kieselgel chromatographisch gereinigt [Laufmittelgradient: Dichlormethan → Dichlormethan-Methanol 7.5 % (v/v)].
LCMS (Methode 2): Rₜ = 3.27 min
MS(ESIpos): m/z = 430 (M+H)⁺

### Beispiel XXI

### 4-(N-Cyclopentylglycyl)-1-isoropyl-2-oxo-1,2,3,4-tetrahydro-6-chinoxalin-carbonsäure-tert-butylester

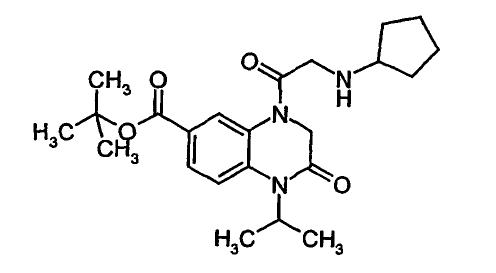

Die Verbindung wird analog Beispiel XX aus den entsprechenden Edukten hergestellt.
LCMS (Methode 7): Rₜ = 3.16 min
MS(ESIpos): m/z = 416 (M+H)⁺

### Beispiel XXII

### 4-(N-Cyclohexylglycyl)-1-cyclopentyl-2-oxo-1,2,3,4-tetrahydro-6-chinoxalin-carbonsäure-tert-butylester

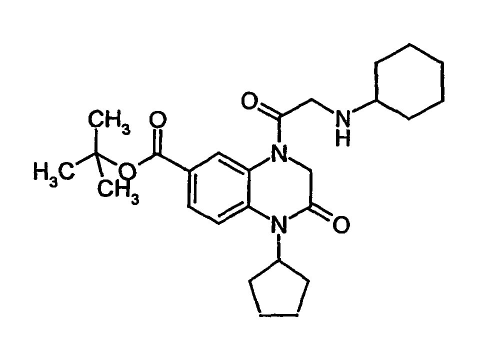

Die Verbindung wird wie für Beispiel XIX beschrieben aus den entsprechenden Edukten hergestellt. Das erhaltene Rohprodukt wird über Kieselgel [Laufmittelgradient: Dichlormethan → Dichlormethan-Methanol 5 % (v/v)] chromatographisch gereinigt. Man erhält ein hellbraunes Öl (410 mg, 21 % d. Th.) als Produkt.
¹H-NMR (400MHz, DMSO-d₆): δ = 0.90-2.10 (m, 27H), 2.31 (m, 1H), 2.42 (m, 1H), 3.53 (br. s, 2H), 4.39 (br. s, 2H), 4.36 (quintett, 2H), 7.43 (d, 1H), 7.79 (d, 1H), 8.08 (br. s, 1H).
MS(ESIpos): m/z = 456 (M+H)⁺

### Beispiel XXIII

### 4-(N-Cyclohexylglycyl)-1-cyclobutyl-2-oxo-1,2,3,4-tetrahydro-6-chinoxalin-carbonsäure-tert-butylester

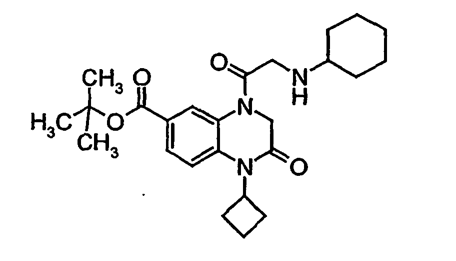

Die Synthese erfolgt wie für die Verbindung in Beispiel XIX beschrieben. Das erhaltene Rohprodukt wird über Kieselgel Laufmittelgradient [Dichlormethan → Dichlormethan-Methanol 5 % (v/v)] chromatographisch gereinigt. Man erhält ein braunes Öl (701 mg, 21 % d. Th.) als Produkt.
¹H-NMR (400MHz, DMSO-d₆): δ = 0.90-1.24 (m, 6H), 1.54 (s, 9H), 1.45-1-82 (m, 8H), 2.00-2.18 (m, 2H), 2.22-2.40 (m, 2H), 3.56 (br. s, 2H), 4.37 (br. s, 2H), 4.45 (quintett, 1H), 7.14 (d, 1H), 7.76 (d, 1H), 8.07 (br. s, 1H).
MS(ESIpos): m/z = 442 (M+H)⁺

### Beispiel XXIV

### 4-(N-Cyclopentylglycyl)-1-cyclopropyl-2-oxo-1,2,3,4-tetrahydro-6-chinoxalin-carbonsäure

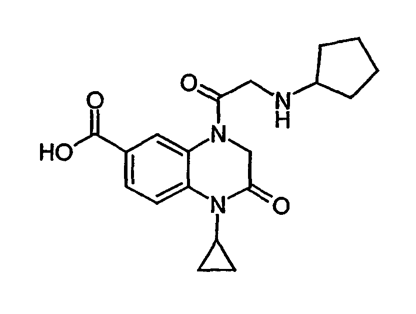

0.11 g (0.27 mmol) der Verbindung aus Beispiel XIX werden mit 2 ml eines Gemisches aus Trifluoressigsäure und Dichlormethan im Verhältnis 1:1 versetzt. Man rührt 30 Minuten bei Raumtemperatur.

Die Lösung wird im Vakuum eingeengt und im Hochvakuum getrocknet. Der Rückstand wird in 10 ml eines Gemisches aus Dichlormethan-Methanol 1:1 aufgenommen und 60 Minuten mit 1 g festem Natriumhydrogencarbonat verrührt. Man verdünnt mit 20 ml Dichlormethan, saugt ab und engt das Filtrat ein. Das erhaltene Produkt wird ohne weitere Reinigung weiter umgesetzt.
LCMS (Methode 3): Rₜ = 0.84 min
MS (ESIpos): m/z = 358 (M+H)⁺

Die in Tabelle 4 aufgeführten Verbindungen werden analog aus den entsprechenden Edukten hergestellt und entweder direkt weiter umgesetzt oder über präp. HPLC (Säulenmaterial: GROM-SIL 120 OSD4 HE, 10 µm; Laufmittelgradient Acetonitril: Wasser 10:90 → 95:5) gereinigt.

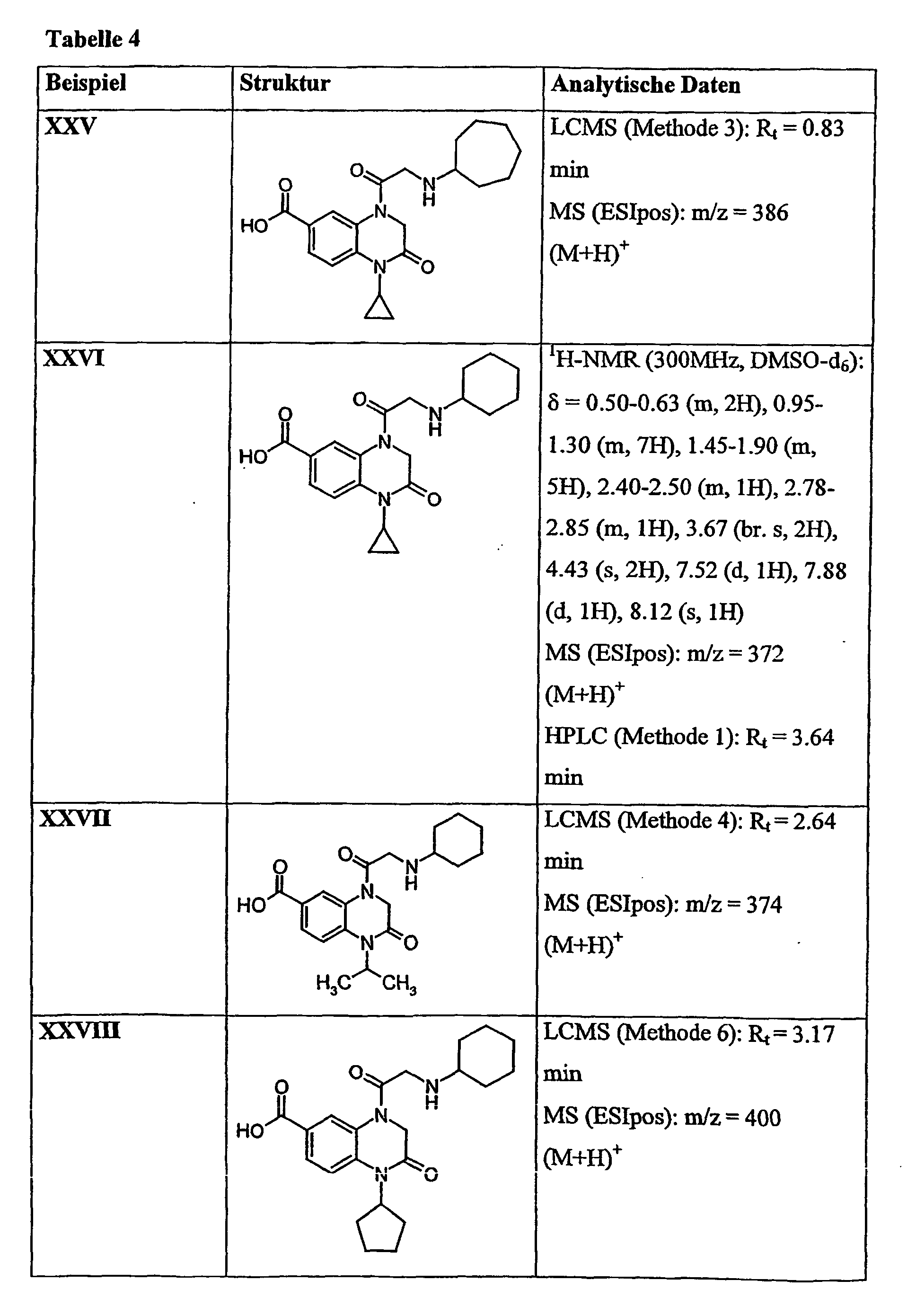

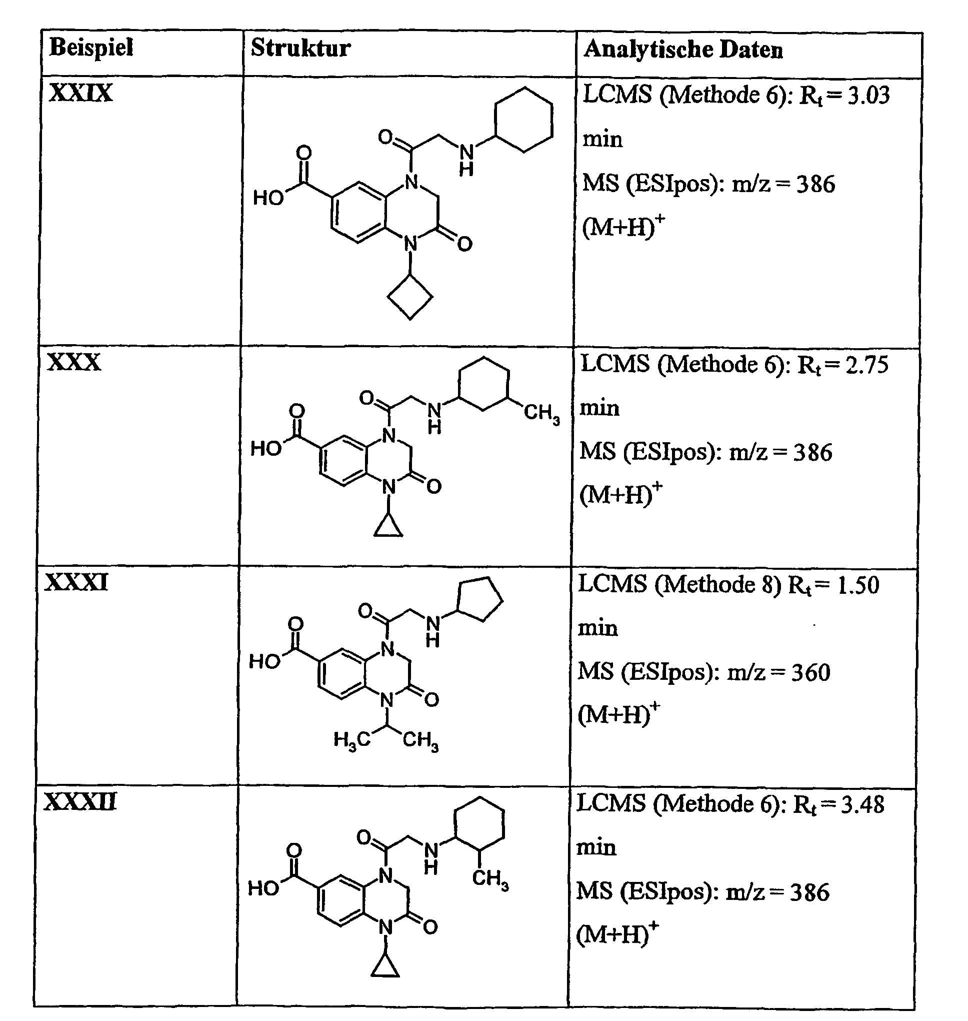

### Beispiel XXXIII

### 4-Chlor-3-nitrobezoesäure-tert-butylester

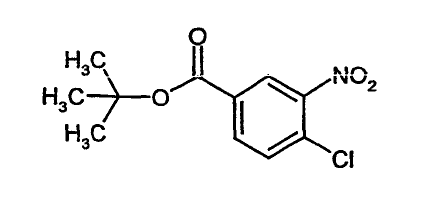

10 g (45.45 mmol) 4-Chlor-3-nitrobenzoesäurechlorid werden in DMF (100 mL) gelöst. 5.10 g Kalium-tert-butylat werden portionsweise bei Raumtemperatur zugegeben. Die Lösung wird eine Stunde bei Raumtemperatur gerührt. Der Ansatz wird dann portionsweise auf Eiswasser (500 mL) gegossen. Der ausfallende Feststoff wird abgesaugt und getrocknet. Man erhält 7.1 g (60% d. Th.) Produkt.
¹H-NMR (300MHz, DMSO-d₆): δ = 1.59 (s, 9H), 7.9 (dd, 1H), 8.18 (m, 1H), 8.49 (dd, 1H)
MS (ESIpos): m/z = 258 (M+H)⁺
HPLC (Methode 1): Rₜ = 5.10 min

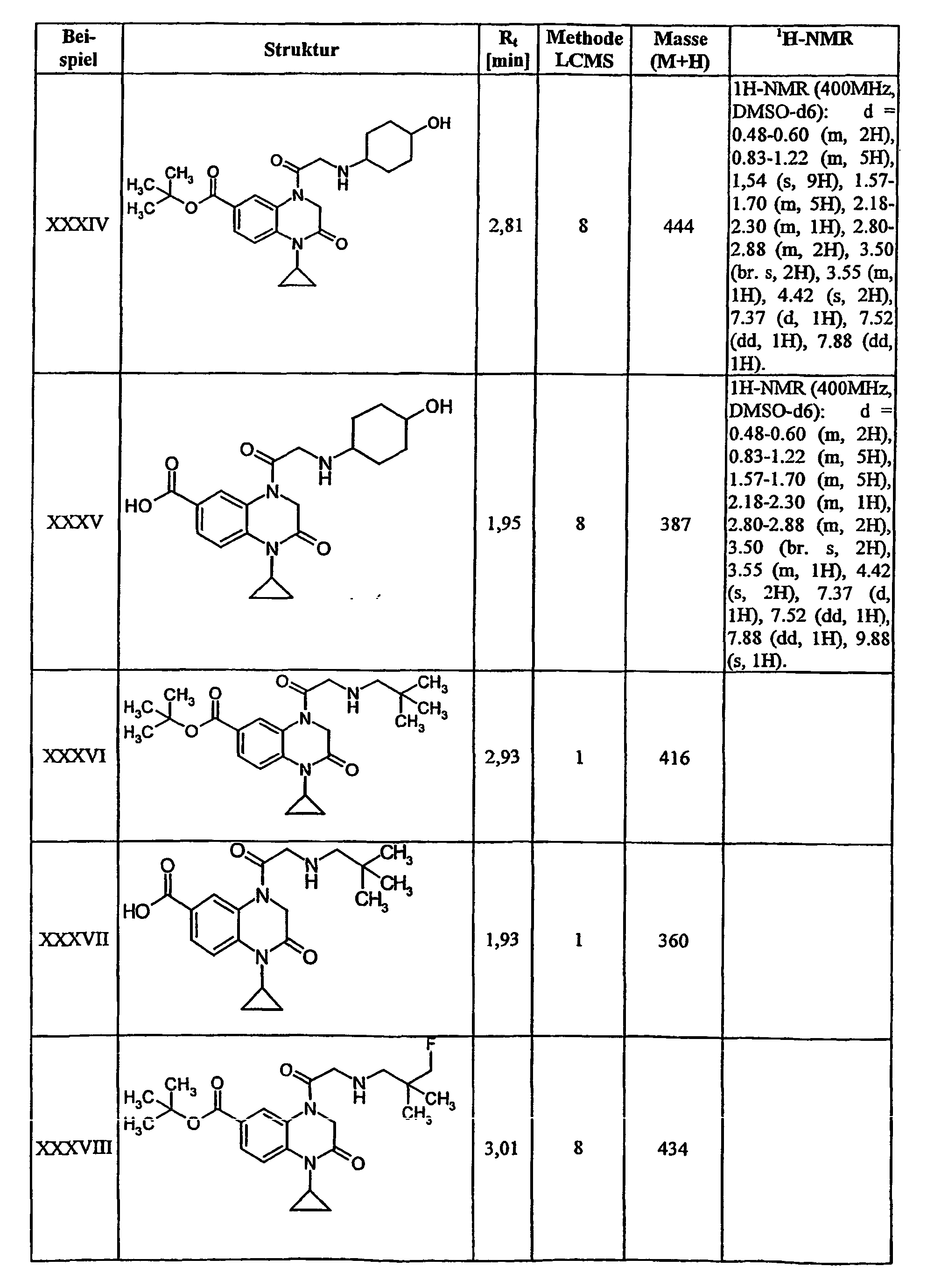

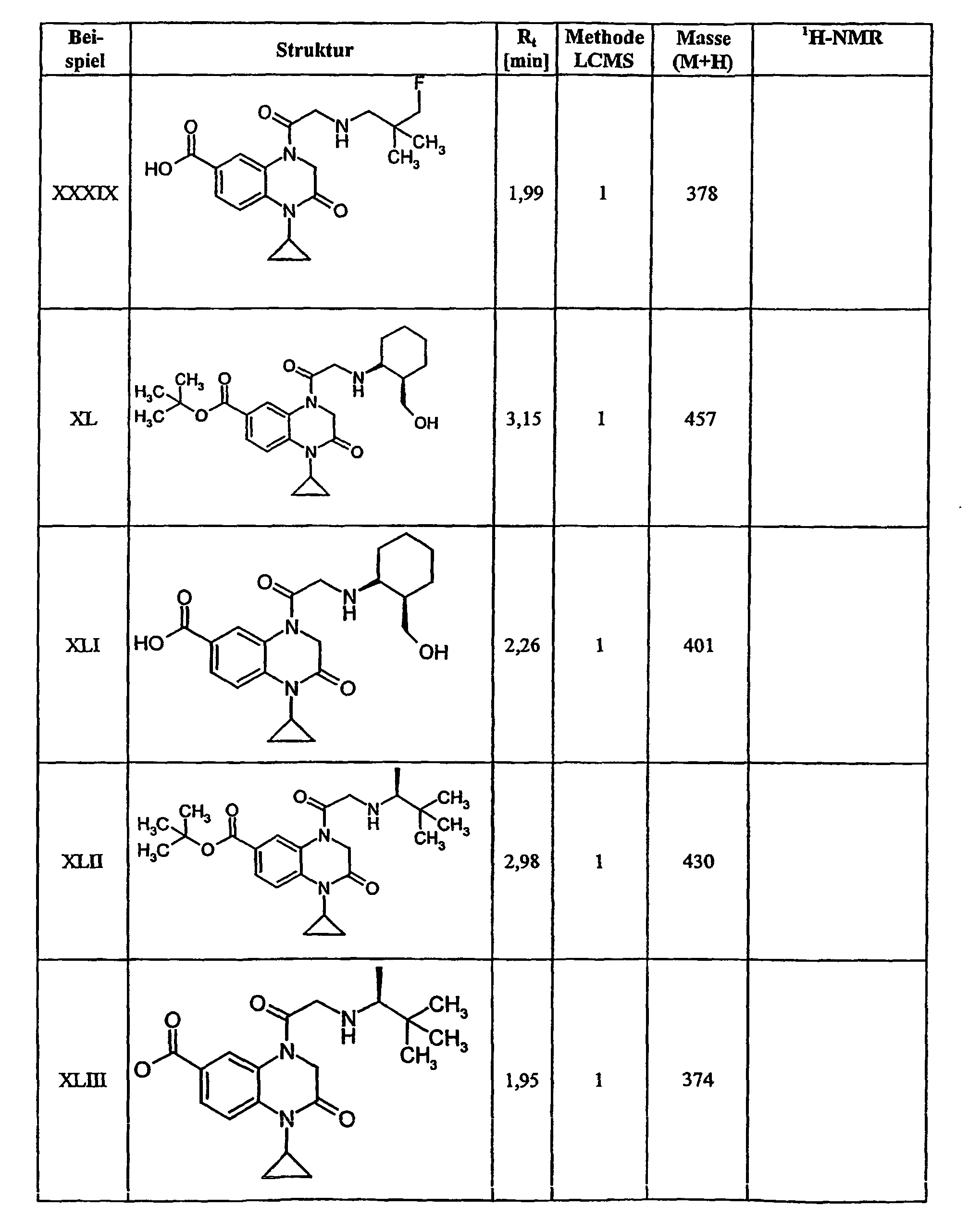

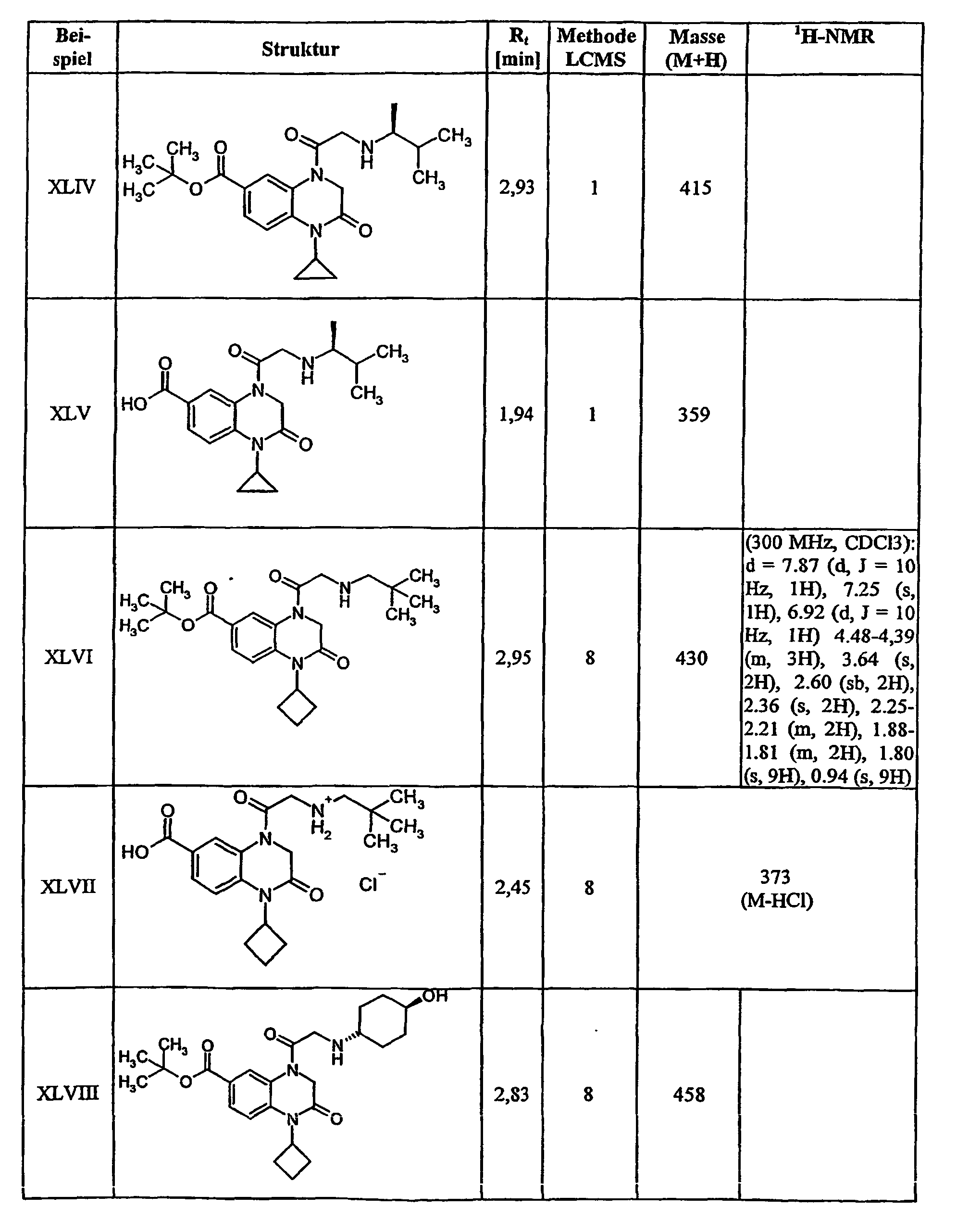

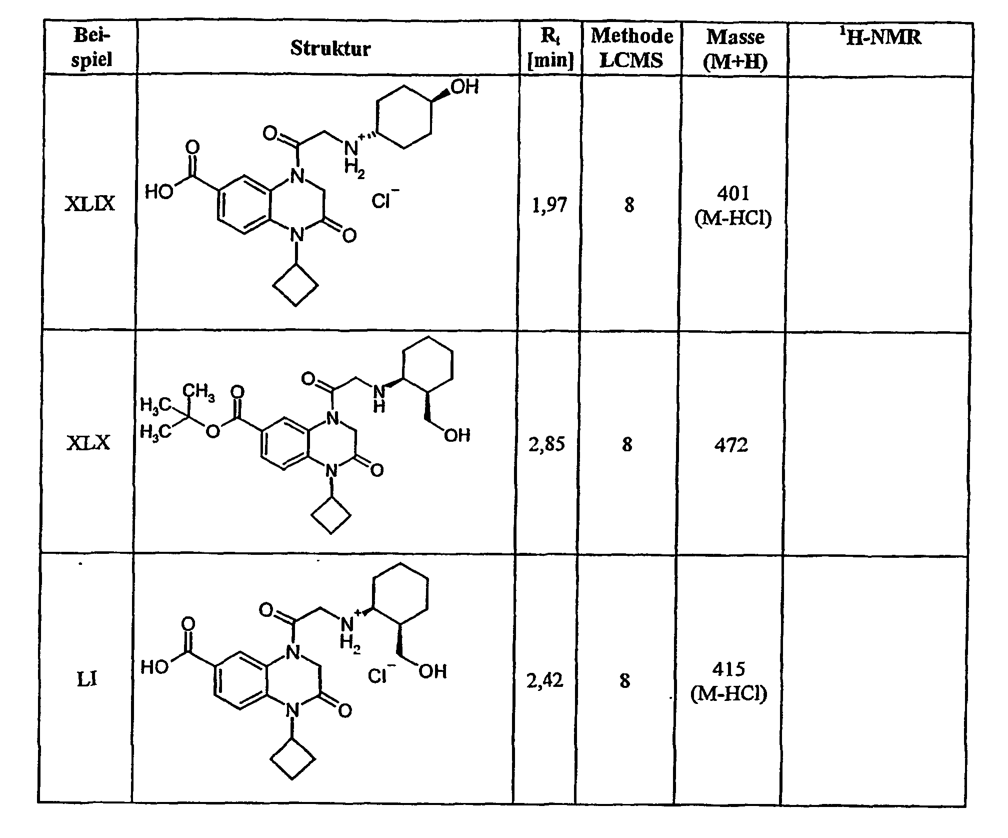

### Ausführungsbeispiele:

### Beispiel 1

### 4-(N-Cyclohexylglycyl)-1-cyclopropyl-N-(3-furylmethyl)-2-oxo-1,2,3,4-tetrahydro-6-chinoxalincarbonsäureamid

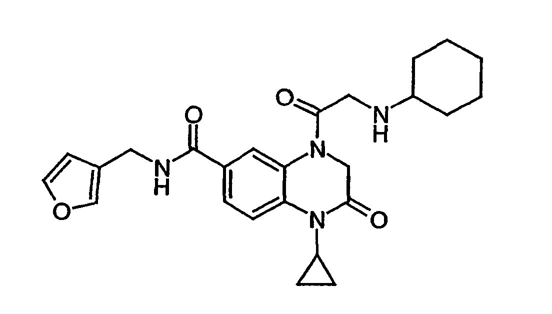

200 mg (0.27 mmol) der Verbindung Beispiel XXV werden in 10 ml Dichlormethan vorgelegt und bei Raumtemperatur mit 36.4 mg (0.27 mmol) 1-Hydroxy-1H-benzotriazol und 51.4 mg (0.27 mmol) 1-(3-Dimethylaminopropyl)-3-ethylcarbodiimid-Hydrochlorid versetzt. Man rührt 20 Minuten und gibt dann 34.8 mg (0.27 mmol) N,N-Düsopropylethylamin und 26.2 mg (0.27 mmol) 3-Aminomethylfuran zu. Es wird 16 Stunden bei Raumtemperatur gerührt.

Das Reaktionsgemisch wird mit 20 ml Dichlormethan versetzt und einmal mit 20 ml Wasser und einmal mit 20 ml gesättigter Natriumchloridlösung gewaschen. Man trocknet über Natriumsulfat und engt im Vakuum ein.

Der Rückstand wird über Kieselgel chromatographisch gereinigt [Laufmittelgradient Dichlormethan → Dichlormethan-Methanol 10% (v/v)].

Man erhält 51 mg (42 % d.Th.) Produkt.
¹H-NMR (300MHz, DMSO-d₆): δ = 0.48-0.57 (m, 2H), 0.79-1.70 (m, 12H), 2.19-2.30 (m, 1H), 2.79-2.87 (m, 1H), 3.51 (s, 2H), 4.31 (d, 2H), 4.41 (s, 2H), 6.45 (s, 1H), 7.50 (d, 1H), 7.58 (s, 2H), 7.83 (d, 1H), 8.02 (s, 1H), 8.80 (t, 1H)
MS (ESIpos): m/z = 451 (M+H)⁺
HPLC (Methode 1): Rₜ = 3.92 min

### Beispiel 2

### 4-(N-Cyclobutylglycyl)-1-cyclopropyl-2-oxo-N-[1-(3-pyridinyl)ethyl]-1,2,3,4-tetrahydro-6-chinoxalincarbonsäureamid

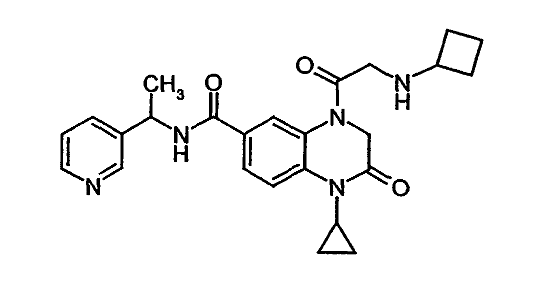

Die Darstellung erfolgt aus den entsprechenden Edukten wie für Beispiel 1 beschrieben.

Man verwendet als Lösungsmittel ein Gemisch aus Dichlormethan-Dimethylformamid im Verhältnis 2:1.
¹H-NMR (300MHz, DMSO-d₆): δ = 0.48-0.57 (m, 2H), 1.02-1.12 (m, 2H), 1.23 (s, 1H), 1.45-1.65 (m, 7H), 1.9-2.0 (m, 2H), 2.79-2.87 (m, 1H), 3.08-3.15 (m, 1H), 3.45 (br. s, 2H), 4.4 (s, 2H), 5.2 (quintett, 1H), 7.35 (dd, 1H), 7.51 (d, 1H), 7.78 (d, 1H), 7.87 (d, 1H), 8.04 (br. s, 1H), 8.44 (dd, 1H), 8.6 (d, 1H), 8.81 (d, 1H)
MS (ESIpos): m/z = 448 (M+H)⁺
HPLC (Methode 1): Rₜ = 3.19 min

### Beispiel 3

### 4-(N-Cyclopentylglycyl)-1-cyclopropyl-2-oxo-N-[1-(4-pyridinyl)ethyl]-1,2,3,4-tetrahydro-6-chinoxalincarbonsäureamid

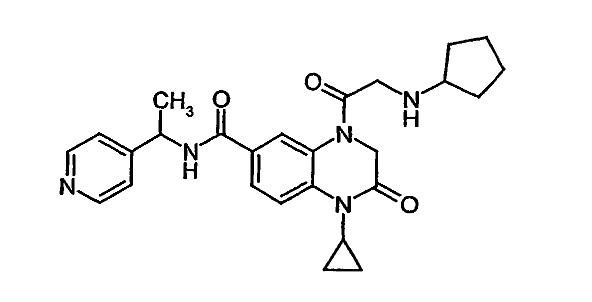

Die Darstellung erfolgt analog der Darstellung der Verbindung aus Beispiel 1 aus den entsprechenden Edukten.

Man verwendet als Lösungsmittel ein Gemisch aus Dichlormethan-Dimethylformamid im Verhältnis 2:1.
¹H-NMR (300MHz, DMSO-d₆): δ = 0.48-0.58 (m, 2H), 1.05-1.3 (m, 4H), 1.33-1.7 (m, 9H), 2.78-3.0 (m, 2H), 3.5 (s, 2H), 4.42 (s, 2H), 5.14 (quintett, 1H), 7.37 (d, 2H), 7.52 (d, 1H), 7.89 (d, 1H), 8.07 (br. s, 1H), 8.50 (d, 2H), 8.83 (d, 1H)
MS (ESIpos): m/z = 462 (M+H)⁺
HPLC (Methode 1): Rₜ = 3.28 min

### Beispiel 4

### 4-(N-Cyclopentylglycyl)-1-cyclopropyl-2-oxo-N-[1-(3-pyridinyl)ethyl]-1,2,3,4-tetrahydro-6-chinoxalincarbonsäureamid

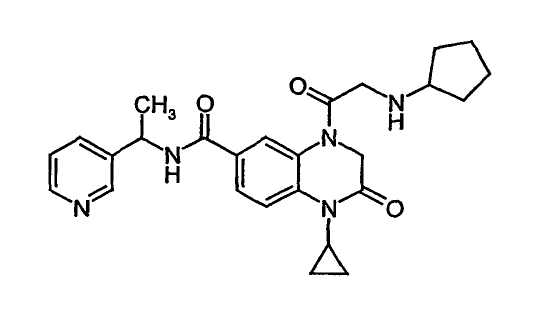

Die Darstellung erfolgt analog der Darstellung der Verbindung aus Beispiel 1 aus den entsprechenden Edukten.

Man verwendet als Lösungsmittel ein Gemisch aus Dichlormethan-Dimethylformamid im Verhältnis 2:1.
¹H-NMR (300MHz, DMSO-d₆): δ = 0.48-0.58 (m, 2H), 1.05-1.25 (m, 5H), 1.33-1.65 (m, 8H), 2.78-3.0 (m, 2H), 3.48 (br. s, 2H), 4.42 (s, 2H), 5.20 (quintett, 1H), 7.35 (dd, 1H), 7.51 (d, 1H), 7.78 (dt, 1H), 7.87 (d, 1H), 8.05 (br. s, 1H), 8.44 (dd, 1H), 8.60 (d, 1H), 8.82 (d, 1H)
MS (ESIpos): m/z = 462 (M+H)⁺
HPLC (Methode 1): Rₜ = 3.28 min

### Beispiel 5

### 4-(N-Cyclohexylglycyl)-1-cyclopropyl-2-oxo-N-[1-(3-pyridinyl)ethyl]-1,2,3,4-tetrahydro-6-chinoxalincarbonsäureamid

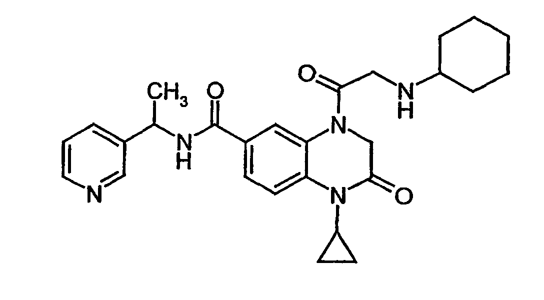

Die Darstellung erfolgt analog der Darstellung der Verbindung aus Beispiel 1 aus den entsprechenden Edukten.

Man verwendet als Lösungsmittel ein Gemisch aus Dichlormethan-Dimethylformamid im Verhältnis 2:1.
¹H-NMR (200MHz, DMSO-d₆): δ = 0.48-0.60 (m, 2H), 0.8-1.25 (m, 8H), 1.40-1.75 (m, 8H), 2.13-2.36 (m, 1H), 2.78-2.9 (m, 1H), 3.51 (br. s, 2H), 4.43 (s, 2H), 5.13 (quintett, 1H), 7.37 (d, 2H), 7.52 (d, 1H), 7.88 (d, 1H), 8.07 (br. s, 1H), 8.50 (d, 2H), 8.89 (d, 1H)
MS (ESIpos): m/z = 476 (M+H)⁺
HPLC (Methode 1): Rₜ = 3.57 min

### Beispiel 6

### 4-(N-Cyclohexylglycyl)-1-cyclopropyl-2-oxo-N-[1-(4-pyridinyl)ethyl]-1,2,3,4-tetrahydro-6-chinoxalincarbonsäureamid

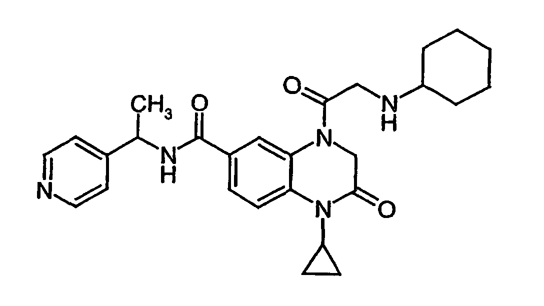

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (400MHz, DMSO-d₆): δ = 0.48-0.60 (m, 2H), 0.83-1.22 (m, 7H), 1.48 (d, 3H), 1.52-1.70 (m, 5H), 2.18-2.30 (m, 1H), 2.80-2.88 (m, 1H), 3.50 (br. s, 2H), 4.42 (s, 2H), 5.13 (quintett, 1H), 7.37 (d, 2H), 7.52 (d, 1H), 7.88 (d, 1H), 8.07 (br. s, 1H), 8.50 (d, 2H), 8.86 (d, 1H)
MS (ESIpos): m/z = 476 (M+H)⁺
HPLC (Methode 1): Rₜ = 3.56 min

### Beispiel 7

### (+)-4-(N-Cyclohexylglycyl)-1-cyclopropyl-2-oxo-N-[1-(4-pyridinyl)ethyl]-1,2,3,4-tetrahydro-6-chinoxalincarbonsäureamid

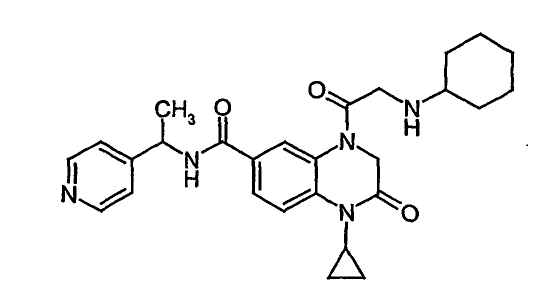

Das Produkt wird durch chromatographische Enantiomerentrennung (Methode 5) der Verbindung aus Beispiel 6 als Fraktion 2 erhalten.
¹H-NMR (400MHz, DMSO-d₆): δ = 0.52 (s, 2H), 0.80-0.98 (m, 2H), 1.02-1.18 (m, 6H), 1.48 (d, 3H), 1.53-1.70 (m, 4H), 1.78-1.90 (m, 1H), 2.18-2.30 (m, 1H), 2.78-2.88 (m, 1H), 3.51 (br. s, 2H), 4.42 (s, 2H), 5.14 (quintett, 1H), 7.36 (d, 2H), 7.51 (d, 1H), 7.87 (d, 1H), 8.07 (br. s, 1H), 8.50 (d, 2H), 8.86 (d, 1H)
MS (ESIpos): m/z = 476 (M+H)⁺
HPLC (Methode 1): Rₜ = 3.41 min
Spezifischer Drehwert: +29.7 ° (Ethanol, T = 20.7°C)

Das zweite Produkt als Fraktion 1 ist: (-)-4-(N-Cyclohexylglycyl)-1-cyclopropyl-2-oxo-N-[1-(4-pyridinyl)ethyl]-1,2,3,4-tetrahydro-6-chinoxalincarbonsäureamid
¹H-NMR (400MHz, DMSO-d₆): δ = 0.52 (s, 2H), 0.80-0.98 (m, 2H), 1.02-1.18 (m, 6H), 1.48 (d, 3H), 1.53-1.70 (m, 4H), 1.78-1.90 (m, 1H), 2.18-2.30 (m, 1H), 2.78-2.88 (m, 1H), 3.51 (br. s, 2H), 4.42 (s, 2H), 5.14 (quintett, 1H), 7.36 (d, 2H), 7.51 (d, 1H), 7.87 (d, 1H), 8.07 (br. s, 1H), 8.50 (d, 2H), 8.86 (d, 1H)
MS (ESIpos): m/z = 476 (M+H)⁺
HPLC (Methode 1): Rₜ = 3.41 min
Spezifischer Drehwert: -36.2 ° (Ethanol, T = 20.9°C)

### Beispiel 8

### 1-Cyclopropyl-4-[N-(2-methylcyclohexyl)glycyl]-2-oxo-N-[1-(4-pyridinyl)ethyl]-1,2,3,4-tetrahydro-6-chinoxalincarbonsäureamid

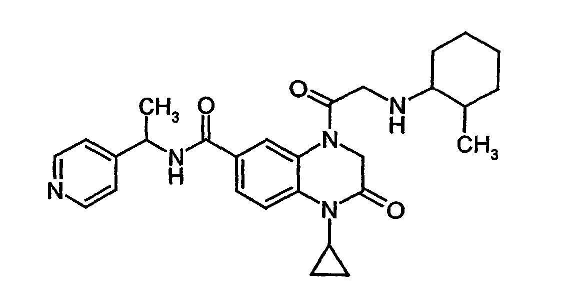

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (300MHz, DMSO-d₆): δ = 0.48-0.60 (m, 2H), 0.76-1.40 (m, 11H), 1.48 (d, 3H), 1.52-1.65 (m, 4H), 1.82-1.95 (m, 1H), 2.80-2.88 (m, 1H), 3.42-3.60 (m, 2H), 4.43 (s, 2H), 5.14 (quintett, 1H), 7.36 (d, 2H), 7.52 (d, 1H), 7.88 (d, 1H), 8.07 (br. s, 1H), 8.49 (d, 2H), 8.83 (d, 1H)
MS (ESIpos): m/z = 490 (M+H)⁺
HPLC (Methode 1): Rₜ = 3.67 min

### Beispiel 9

### 1-Cyclopropyl-4-[N-(2-methylcyclohexyl)glycyl]-2-oxo-N-[1-(3-pyridinyl)ethyl]-1,2,3,4-tetrahydro-6-chinoxalincarbonsäureamid

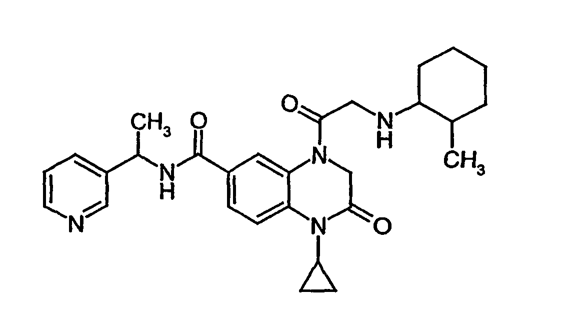

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (300MHz, DMSO-d₆): δ = 0.58-0.68 (m, 2H), 0.72-1.35 (m, 13H), 1.51 (d, 3H), 1.52-1.65 (m, 1H), 1.82-1.95 (m, 1H), 2.76-2.88 (m, 1H), 3.42-3.60 (m, 2H), 4.43 (s, 2H), 5.20 (quintett, 1H), 7.35 (dd, 2H), 7.51 (d, 1H), 7.78 (d, 1H), 7.85 (d, 1H), 8.06 (br. s, 1H), 8.44 (dd, 1H), 8.60 (d, 1H), 8.81 (d, 1H)
MS (ESIpos): m/z = 490 (M+H)⁺
HPLC (Methode 1): Rₜ = 3.68 min

### Beispiel 10

### 1-Cyclopropyl-4-[N-(3-methylcyclohexyl)glycyl]-2-oxo-N-[1-(4-pyridinyl)ethyl]-1,2,3,4-tetrahydro-6-chinoxalincarbonsäureamid

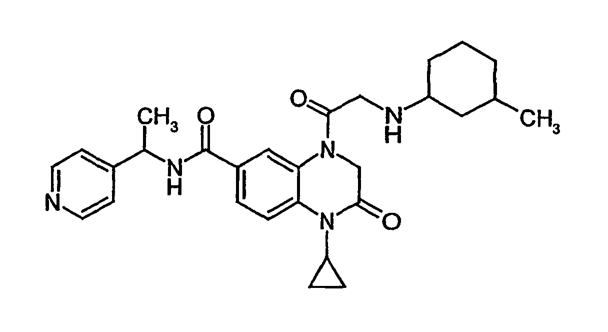

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (300MHz, DMSO-d₆): δ = 0.48-0.58 (m, 2H), 0.68-0.95 (m, 5H), 1.02-1.38 (m, 6H), 1.48 (d, 3H), 1.45-1.72 (m, 4H), 2.20-2.32 (m, 1H), 2.79-2.88 (m, 1H), 3.42-3.58 (m, 2H), 4.42 (s, 2H), 5.14 (quintett, 1H), 7.36 (d, 2H), 7.51 (d, 1H), 7.88 (d, 1H), 8.08 (br. s, 1H), 8.50 (dd, 2H), 8.83 (d, 1H)
MS (ESIpos): m/z = 490 (M+H)⁺
HPLC (Methode 1): Rₜ = 3.71 min

### Beispiel 11

### 1-Cyclopropyl-4-[N-(3-methylcyclohexyl)glycyl]-2-oxo-N-[1-(3-pyridinyl)ethyl]-1,2,3,4-tetrahydro-6-chinoxalincarbonsäureamid

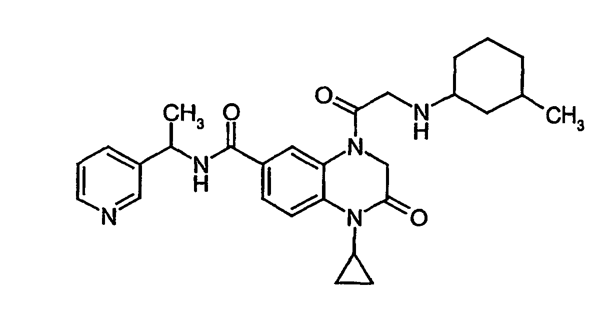

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (300MHz, DMSO-d₆): δ = 0.48-0.58 (m, 2H), 0.68-0.95 (m, 5H), 1.02-1.38 (m, 6H), 1.51 (d, 3H), 1.53-1.75 (m, 4H), 2.20-2.32 (m, 1H), 2.79-2.88 (m, 1H), 3.42-3.58 (m, 2H), 4.41 (s, 2H), 5.20 (quintett, 1H), 7.34 (dd, 1H), 7.51 (d, 1H), 7.78 (d, 1H), 7.86 (d, 1H), 8.06 (br. s, 1H), 8.44 (dd, 1H), 8.60 (d, 1H), 8.81 (d, 1H)
MS (ESIpos): m/z = 490 (M+H)⁺
HPLC (Methode 1): Rₜ = 3.72 min

### Beispiel 12

### 4-(N-Cyclohexylglycyl)-1-cyclopropyl-2-oxo-N-(3-thienylmethyl)-1,2,3,4-tetrahydro 6-chinoxalincarbonsäureamid

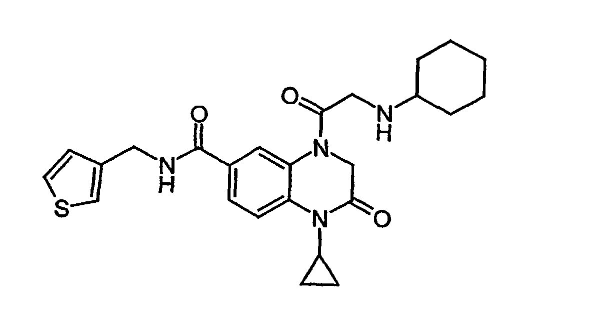

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (300MHz, DMSO-d₆): δ = 0.48-0.58 (m, 2H), 0.82-0.95 (m, 2H), 1.05-1.18 (m, 5H), 1.23 (s, 1H), 1.45-1.72 (m, 5H), 2.20-2.32 (m, 1H), 2.79-2.88 (m, 1H), 3.51 (s, 2H), 4.42 (s, 2H), 4.47 (d, 2H), 7.08 (dd, 1H), 7.29-7.33 (m, 1H), 7.45-7.50 (m, 1H), 7.52 (s, 1H), 7.85 (d, 1H), 8.04 (br. s, 1H), 8.93 (t, 1H)
MS (ESIpos): m/z = 467 (M+H)⁺
HPLC (Methode 1): Rₜ = 4.05 min

### Beispiel 13

### 4-(N-Cyclohexylglycyl)-1-isopropyl-2-oxo-N-[1-(4-pyridinyl)ethyl]-1,2,3,4-tetrahydro-6-chinoxalincarbonsäureamid

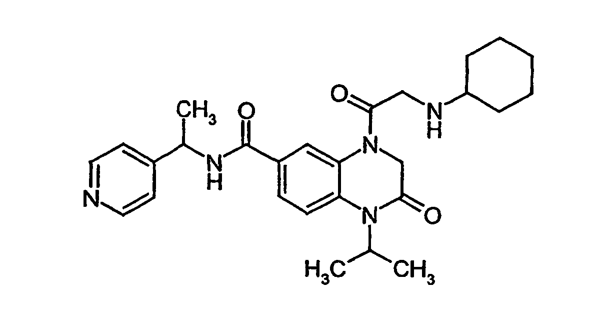

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 5:1 verwendet.
¹H-NMR (200MHz, DMSO-d₆): δ = 0.70-1.85 (m, 12H), 1.17 (d, 3H), 1.47 (t, 6H), 3.54 (br. s, 2H), 4.37 (s, 2H), 4.60 (quintett, 1H), 5.14 (quintett, 1H), 7.36 (d, 2H), 7.50 (d, 1H), 7.85 (d, 1H), 8.10 (br. s, 1H), 8.50 (d, 2H), 8.92 (d, 1H)
MS (ESIpos): m/z = 478 (M+H)⁺
HPLC (Methode 1): Rₜ = 3.63 min

### Beispiel 14

### 4-(N-Cycloheptylglycyl)-1-cyclopropyl-2-oxo-N-[1-(4-pyridinyl)ethyl]-1,2,3,4-tetrahydro-6-chinoxalincarbonsäureamid

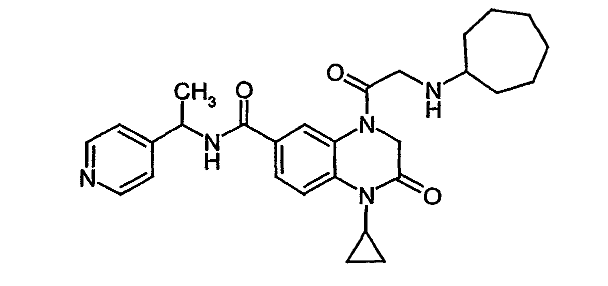

Die Darstellung erfolgt analog der Darstellung der Verbindung aus Beispiel 1 aus den entsprechenden Edukten.
¹H-NMR (400MHz, DMSO-d₆): δ = 0.53 (s, 2H), 1.03-1.65 (m, 16H), 1.48 (d, 3H), 2.79-2.87 (m, 1H), 3.49 (br. s, 2H), 4.42 (s, 2H), 5.14 (quintett, 1H), 7.36 (d, 2H), 7.52 (d, 1H), 7.88 (d, 1H), 8.08 (br. s, 1H), 8.50 (d, 2H), 8.86 (d, 1H)
MS (ESIpos): m/z = 490 (M+H)⁺
HPLC (Methode 1): Rₜ = 3.58 min

### Beispiel 15

### 4-(N-Cycloheptylglycyl)-1-cyclopropyl-2-oxo-N-[1-(3-pyridinyl)ethyl]-1,2,3,4-tetrahydro-6-chinoxalincarbonsäureamid

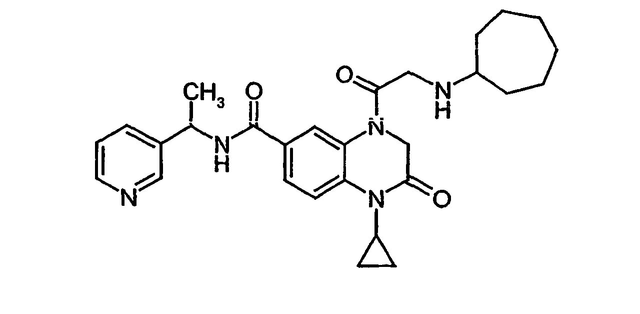

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (400MHz, DMSO-d₆): δ = 0.52 (s, 2H), 1.03-1.88 (m, 16H), 1.51 (d, 3H), 2.80-2.85 (m, 1H), 3.47 (br. s, 2H), 4.41 (s, 2H), 5.19 (quintett, 1H), 7.34 (dd, 1H), 7.65 (dd, 2H), 7.85 (d, 1H), 8.06 (br. s, 1H), 8.63 (dd, 2H), 8.60 (d, 1H)
MS (ESIpos): m/z = 490 (M+H)⁺
HPLC (Methode 1): Rₜ = 3.58 min

### Beispiel 16

### 4-(N-Cyclohexylglycyl)-1-cyclopentyl-2-oxo-N-[1-(3-pyridinyl)ethyl]-1,2,3,4-tetrahydro-6-chinoxalincarbonsäureamid

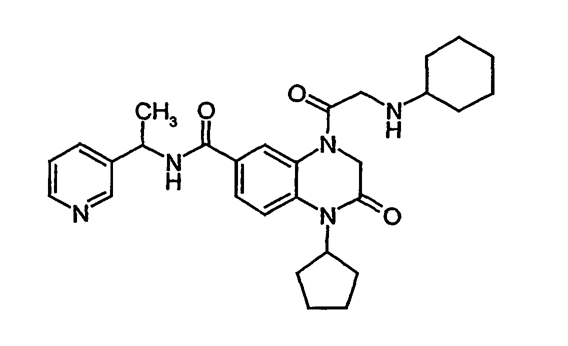

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (400MHz, DMSO-d₆): δ = 0.82-1.20 (m, 6H), 1.51 (d, 3H), 1.53-2.05 (m, 13H), 2.25-2.38 (m, 1H), 3.58 (s, 2H), 4.38 (br. s, 2H), 4.63 (quintett, 1H), 5.20 (quintett, 1H), 7.34 (dd, 1H), 7.60 (dd, 2H), 7.78 (d, 1H), 8.09 (br. s, 1H), 8.44 (d, 1H), 8.60 (d, 1H), 8.86 (d, 1H)
MS (ESIpos): m/z = 504 (M+H)⁺
HPLC (Methode 1): Rₜ = 3.79 min

### Beispiel 17

### 1-Cyclobutyl-4-(N-cyclohexylglycyl)-2-oxo-N-[1-(4-pyridinyl)ethyl]-1,2,3,4-tetrahydro-6-chinoxalincarbonsäureamid

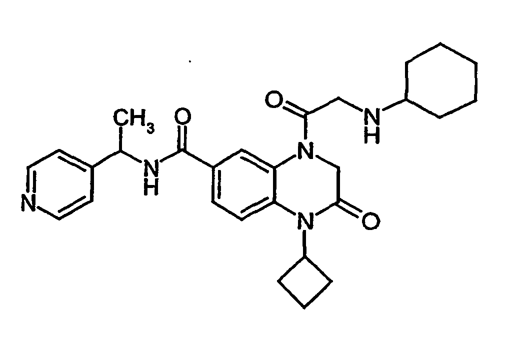

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (400MHz, DMSO-d₆): δ = 0.80-1.13 (m, 8H), 1.48 (d, 3H), 1.49-2.12 (m, 9H), 2.25-2.33 (m, 1H), 3.55 (br. s, 2H), 4.30-4.50 (m, 3H), 5.13 (quintett, 1H), 7.13 (d, 1H), 7.81 (d, 1H), 7.90 (dd, 4H), 8.08 (br. s, 1H), 8.87 (d, 1H)
MS (ESIpos): m/z = 490 (M+H)⁺
HPLC (Methode 1): Rₜ = 3.64 min

### Beispiel 18

### 1-Cyclobutyl-4-(N-cyclohexylglycyl)-2-oxo-N-[1-(3-pyridinyl)ethyl]-1,2,3,4-tetrahydro-6-chinoxalincarbonsäureamid

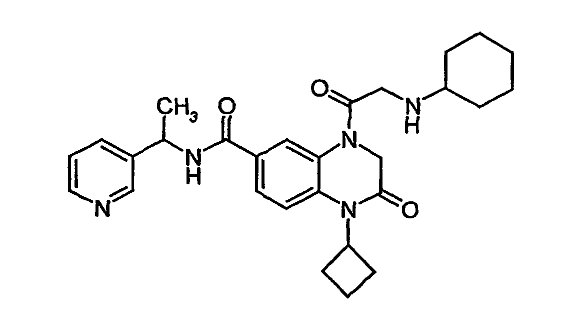

Die Darstellung erfolgt analog der Darstellung der Verbindung aus Beispiel 1 aus den entsprechenden Edukten.

Als Lösungsmittel wird ein Gemisch aus Dichlormethan-Dimethylformamid im Verhältnis 2:1 verwendet.
¹H-NMR (400MHz, DMSO-d₆): δ = 0.80-1.30 (m, 6H), 1.51 (d, 3H), 1.52-1.92 (m, 9H), 2.00-2.18 (m, 2H), 2.22-2.36 (m, 1H), 3.55 (br. s, 2H), 4.37 (br. s, 2H), 4.45 (quintett, 1H), 5.19 (quintett, 1H), 7.13 (d, 1H), 7.32-7.37 (m, 1H), 7.77-7.81 (m, 2H), 8.07 (br. s, 1H), 8.84 (d, 1H), 8.60 (d, 1H), 8.85 (d, 1H)
MS (ESIpos): m/z = 490 (M+H)⁺
HPLC (Methode 1): Rₜ = 3.63 min

### Beispiel 19

### 4-(N-Cyclohexylglycyl)-1-cyclopropyl-N-[1-(6-methyl-3-pyridinyl)ethyl]-2-oxo-1,2,3,4-tetrahydro-6-chinoxalincarbonsäureamid

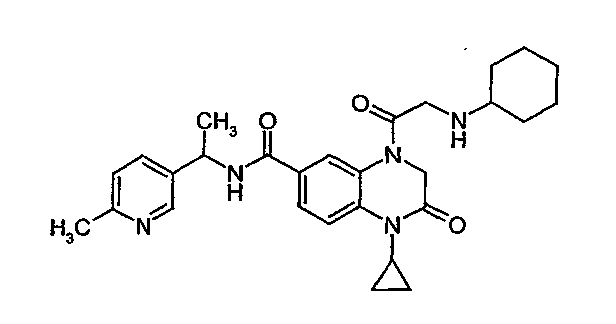

Die Darstellung erfolgt analog der Darstellung der Verbindung aus Beispiel 1 aus den entsprechenden Edukten.
¹H-NMR (400MHz, DMSO-d₆): δ = 0.52 (s, 2H), 0.80-1.20 (m, 8H), 1.49 (d, 3H), 1.52-1.92 (m, 5H), 2.15-2.30 (m, 1H), 2.43 (s, 3H), 2.82 (s, 1H), 3.49 (br. s, 2H), 4.42 (s, 2H), 5.15 (quintett, 1H), 7.19 (d, 1H), 7.50 (d, 1H), 7.66 (d, 1H), 7.85 (d, 1H), 8.04 (br. s, 1H), 8.45 (s, 1H), 8.78 (d, 1H)
MS (ESIpos): m/z = 490 (M+H)⁺
HPLC (Methode 1): Rₜ = 3.50 min

### Beispiel 20

### 4-(N-Cyclohexylglycyl)-1-cyclopropyl-N-[1-(6-methoxy-3-pyridinyl)ethyl]-2-oxo-1,2,3,4-tetrahydro-6-chinoxalincarbonsäureamid

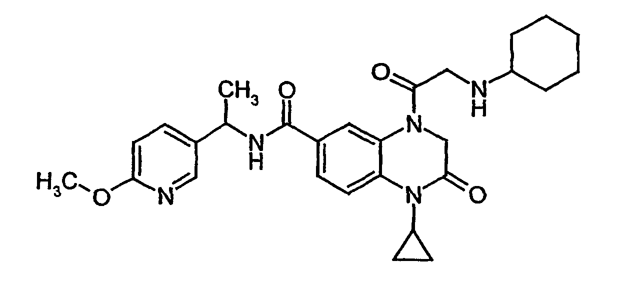

Die Darstellung erfolgt analog der Darstellung der Verbindung aus Beispiel 1 aus den entsprechenden Edukten.
¹H-NMR (400MHz, DMSO-d₆): δ = 0.51 (s, 2H), 0.80-0.98 (m, 2H), 1.00-1.20 (m, 6H), 1.48 (d, 3H), 1.52-1.89 (m, 5H), 2.15-2.30 (m, 1H), 2.82 (s, 1H), 3.49 (br. s, 2H), 3.81 (s, 3H), 4.41 (s, 2H), 5.14 (quintett, 1H), 6.78 (d, 1H), 7.49 (d, 1H), 7.73 (dd, 1H), 7.83 (d, 1H), 8.04 (br. s, 1H), 8.16 (d, 1H), 8.75 (d, 1H)
MS (ESIpos): m/z = 506 (M+H)⁺
HPLC (Methode 1): Rₜ = 3.58 min

### Beispiel 21

### 4-(N-Cyclohexylglycyl)-1-cyclopropyl-2-oxo-N-(3-pyridinylmethyl)-1,2,3,4-tetrahydro-6-chinoxalincarbonsäureamid

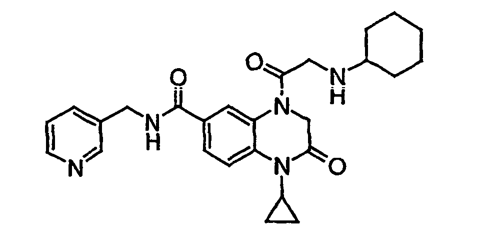

Die Darstellung erfolgt analog der Darstellung der Verbindung aus Beispiel 1 aus den entsprechenden Edukten.

Das Rohprodukt wird über präp. HPLC [Säulenmaterial: GROM-SIL 120 OSD4 HE, 10 µm, Laufmittelgradient Acetonitril/Wasser 10 : 90 → 95 : 5 (v/v)] chromatographisch gereinigt.
¹H-NMR (300MHz, DMSO-d₆): δ = 0.53 (m, 2H), 0.79-1.28 (m, 6H), 1.40-1.76 (m, 6H), 2.26 (m, 1H), 2.82 (m, 1H), 3.51 (s, 2H), 4.42 (s, 2H), 4.51 (d, 2H), 7.35 (dd, 1H), 7.52 (d, 1H), 7.72 (dt, 1H), 7.86 (d, 1H), 8.05 (br. s, 1H), 8.45 (dd, 1H), 8.56 (d, 1H), 9.05 (dd, 1H)
MS (ESIpos): m/z = 462 (M+H)⁺

### Beispiel 22

### 4-N-[(trans-4-Cyclohexanolyl)glycyl]-1-cyclopropyl-2-oxo-N-[1-S-(4-pyridinyl)-ethyl]-1,2,3,4-tetrahydro-6-chinoxalincarbonsäureamid

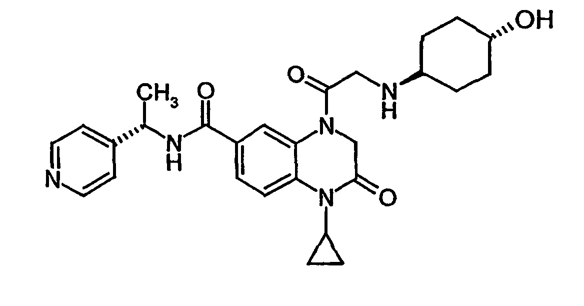

Die Darstellung erfolgt analog der Darstellung der Verbindung aus Beispiel 1 aus den entsprechenden Edukten. Der Rückstand wird mittels präparativer HPLC (Acetonitril-Wasser) gereinigt.
¹H-NMR (400MHz, DMSO-d₆): δ = 0.48-0.60 (m, 2H), 0.83-1.22 (m, 5H), 1.48 (d, 3H), 1.52-1.70 (m, 5H), 2.18-2.30 (m, 1H), 2.80-2.88 (m, 2H), 3.50 (br. s, 2H), 3.55 (m, 1H), 4.42 (s, 2H), 5.13 (quintett, 1H), 7.37 (d, 2H), 7.52 (d, 1H), 7.88 (d, 1H), 8.07 (br. s, 1H), 8.50 (d, 2H), 8.86 (d, 1H)
MS (ESIpos): m/z = 492 (M+H)⁺
HPLC (Methode 1): Rₜ = 3.43 min

### Beispiel 23

### 4-N-[(2,2-Dimethylbutyl)glycyl]-1-cyclopropyl-2-oxo-N-[1-S-(4-pyridinyl)ethyl]-1,2,3,4-tetrahydro-6-chinoxalincarbonsäureamid

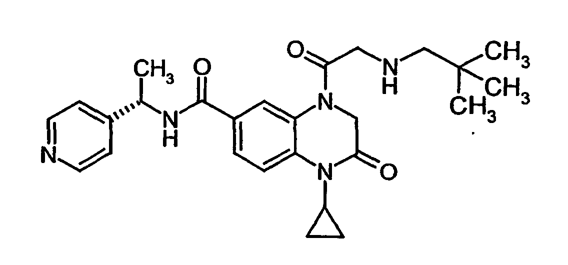

Die Darstellung erfolgt analog der Darstellung der Verbindung aus Beispiel 1 aus den entsprechenden Edukten. Der Rückstand wird mittels präparativer HPLC (Acetonitril-Wasser) gereinigt.
¹H-NMR (400MHz, DMSO-d₆): δ = 0.56 (m, 2H), 0.82 (s, 9H), 1.11 (m, 2H), 1.48 (d, 3H), 2.25 (br. s, 1H), 2.85 (m, 2H), 3.50 (s, 2H), 4.42 (s, 2H), 5.13 (quintett, 1H), 7.37 (d, 2H), 7.51 (d, 1H), 7.88 (d, 1H), 8.07 (br. s, 1H), 8.50 (d, 2H), 8.86 (d, 1H)
MS (ESIpos): m/z = 464 (M+H)⁺
HPLC (Methode 1): Rₜ = 3.42 min

### Beispiel 24

### 4-N-[(1-S-Methyl-2,2-dimethylbutyl)glycyl]-1-cyclopropyl-2-oxo-N-[1-S-(4-pyridinyl)ethyl]-1,2,3,4-tetrahydro-6-chinoxalincarbonsäureamid

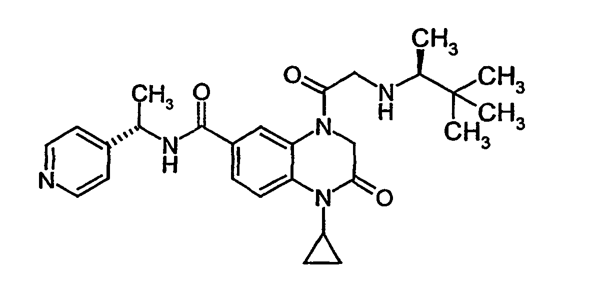

Die Darstellung erfolgt analog der Darstellung der Verbindung aus Beispiel 1 aus den entsprechenden Edukten. Der Rückstand wird mittels präparativer HPLC (Acetonitril-Wasser) gereinigt.
¹H-NMR (400MHz, DMSO-d₆): δ = 0.56 (m, 2H), 0.78 (s, 9H), 1.11 (m, 2H), 1.49 (d, 3H), 2.12 (d, 3H), 2.25 (br. s, 1H), 2.85 (m, 1H), 2.90 (m, 1H), 3.50 (s, 2H), 4.42 (s, 2H), 5.13 (quintett, 1H), 7.37 (d, 2H), 7.51 (d, 1H), 7.88 (d, 1H), 8.07 (br. s, 1H), 8.50 (d, 2H), 8.86 (d, 1H)
MS (ESIpos): m/z = 478 (M+H)⁺
HPLC (Methode 1): Rₜ = 3.53 min

### Beispiel 25

### 4-N-[1-S-Methyl-(2-methylbutyl)glycyl]-1-cyclopropyl-2-oxo-N-[1-S-(4-pyridinyl)-ethyl]-1,2,3,4-tetrahydro-6-chinoxalincarbonsäureamid

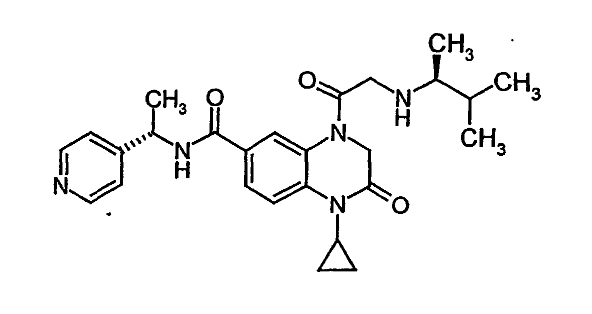

Die Darstellung erfolgt analog der Darstellung der Verbindung aus Beispiel 1 aus den entsprechenden Edukten. Der Rückstand wird mittels präparativer HPLC (Acetonitril-Wasser) gereinigt.
¹H-NMR (400MHz, DMSO-d₆): δ = 0.56 (m, 2H), 0.74 (d, 6H), 1.11 (m, 2H), 1,25 (m, 1H) 1.49 (d, 3H), 2.12 (d, 3H), 2.22 (br. s, 1H), 2.85 (m, 1H), 2.90 (m, 1H), 3.50 (s, 2H), 4.42 (s, 2H), 5.13 (quintett, 1H), 7.37 (d, 2H), 7.51 (d, 1H), 7.88 (d, 1H), 8.07 (br. s, 1H), 8.50 (d, 2H), 8.86 (d, 1H)
MS (ESIpos): m/z = 464 (M+H)⁺
HPLC (Methode 1): Rₜ = 3.40 min

### Beispiel 26

### 4-N-[1-S-Methyl-(2-methylbutyl)glycyl]-1-cyclopropyl-2-oxo-N-[(3-pyridinyl-4-methoxy)ethyl]-1,2,3,4-tetrahydro-6-chinoxalincarbonsäureamid

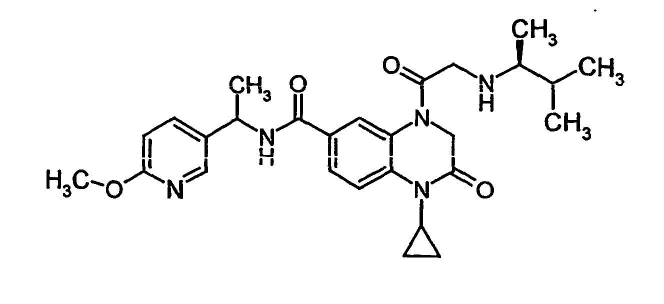

Die Darstellung erfolgt analog der Darstellung der Verbindung aus Beispiel 1 aus den entsprechenden Edukten. Der Rückstand wird mittels präparativer HPLC (Acetonitril-Wasser) gereinigt.
¹H-NMR (400MHz, DMSO-d₆): δ = 0.57 (m, 2H), 0.73 (d, 6H), 1.12 (m, 2H), 1,25 (m, 1H) 1.49 (d, 3H), 2.15 (d, 3H), 2.22 (br. s, 1H), 2.85 (m, 1H), 2.90 (m, 1H), 3,23 (s, 3H), 3.50 (s, 2H), 4.42 (s, 2H), 5.06 (quintett, 1H), 7.49 (d, 2H), 7.52 (m, 1H), 7.83 (d, 1H), 8.20 (d, 1H), 8.85 (d, 1H)
MS (ESIpos): m/z = 464 (M+H)⁺
HPLC (Methode 1): Rₜ = 3.40 min

### Beispiel 27

### 4-N-{[2,2-Dimethyl-(3-fluor)butyl]glycyl}-1-cyclopropyl-2-oxo-N-[1-S-(4-pyridinyl)ethyl]-1,2,3,4-tetrahydro-6-chinoxalincarbonsäureamid

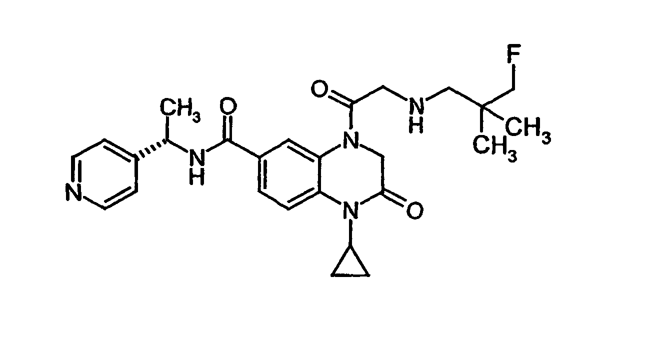

Die Darstellung erfolgt analog der Darstellung der Verbindung aus Beispiel 1 aus den entsprechenden Edukten. Der Rückstand wird mittels präparativer HPLC (Acetonitril-Wasser) gereinigt.
¹H-NMR (400MHz, DMSO-d₆): δ = 0.56 (m, 2H), 1.10 (m, 8H), 1.61 (d, 3H), 2.90 (m, 4H), 4.15 (s, 1H), 4.42 (s, 2H), 5.13 (m, 1H), 7.37 (d, 2H), 7.51 (d, 1H), 7.88 (d, 1H), 8.07 (br. s, 1H), 8.50 (d, 2H), 8.86 (d, 1H)
MS (ESIpos): m/z = 482 (M+H)⁺
HPLC (Methode 1): Rₜ = 3.35 min

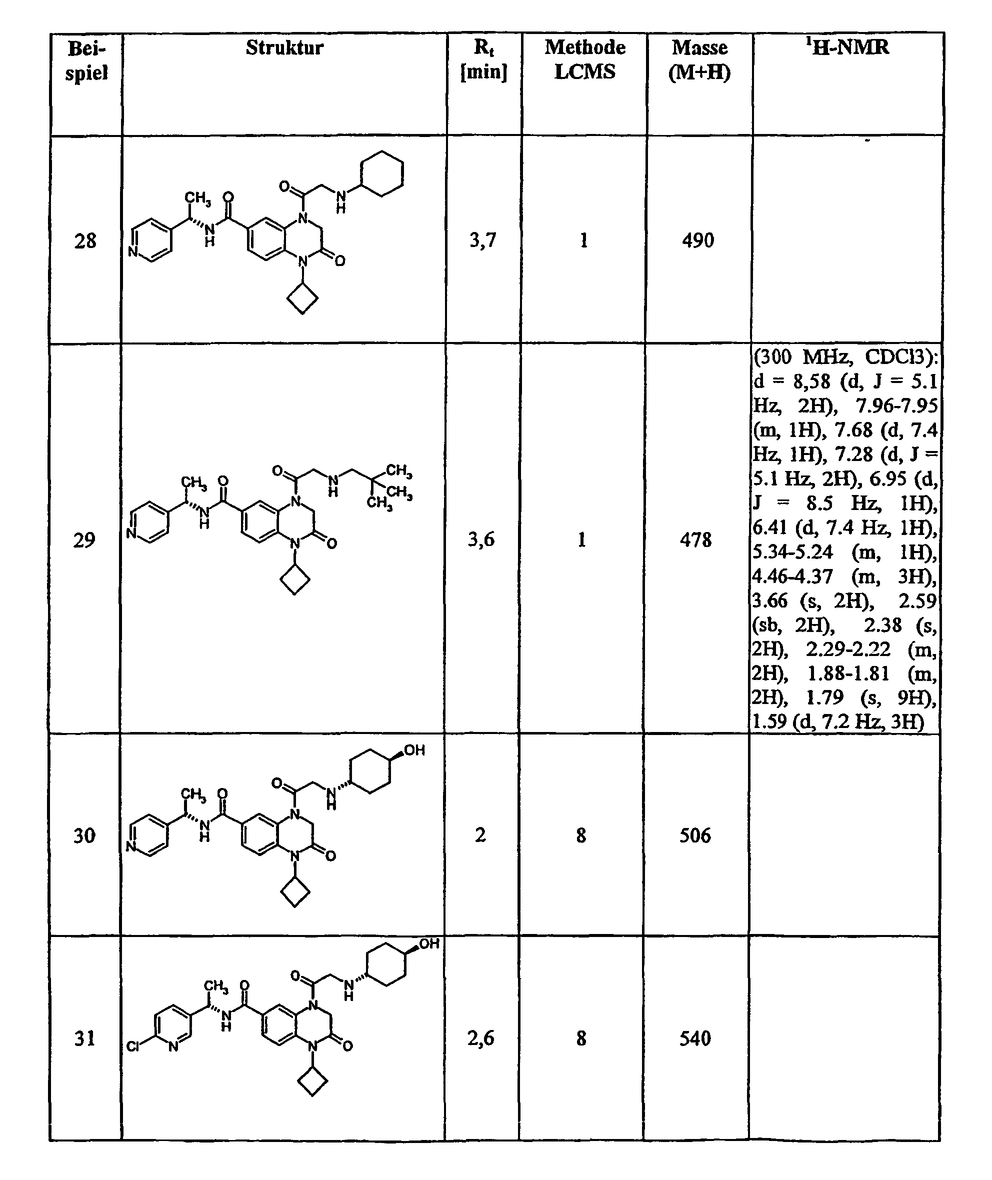

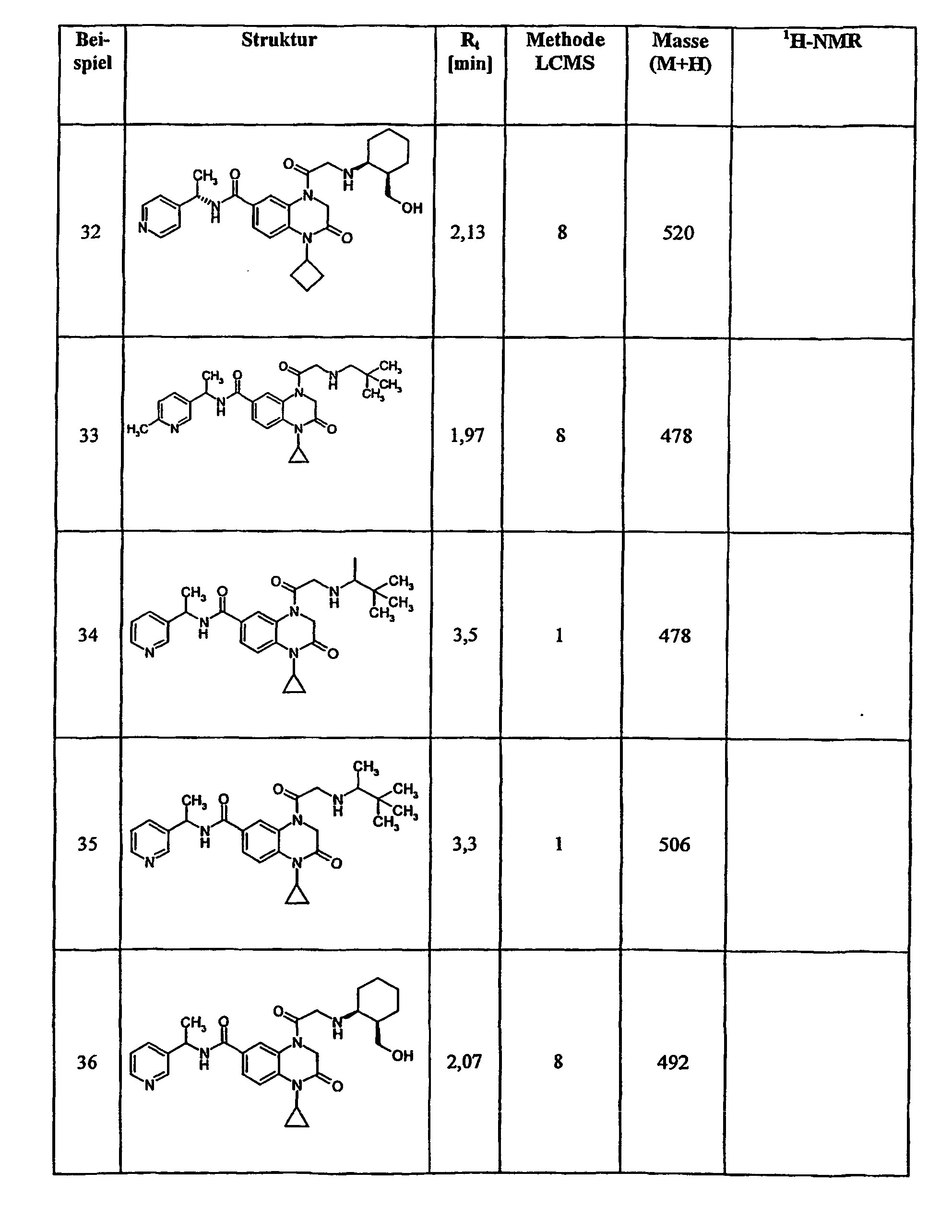

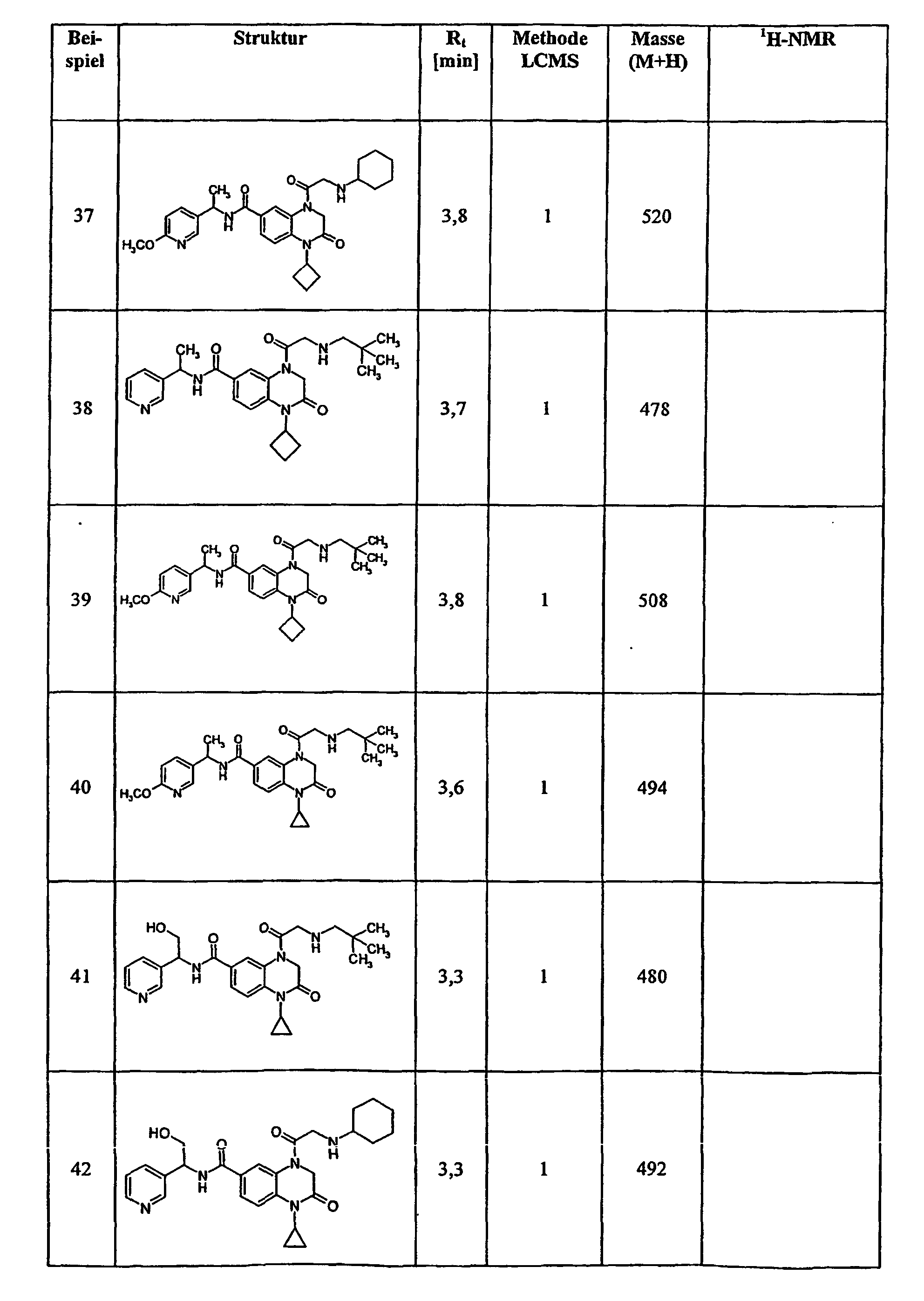

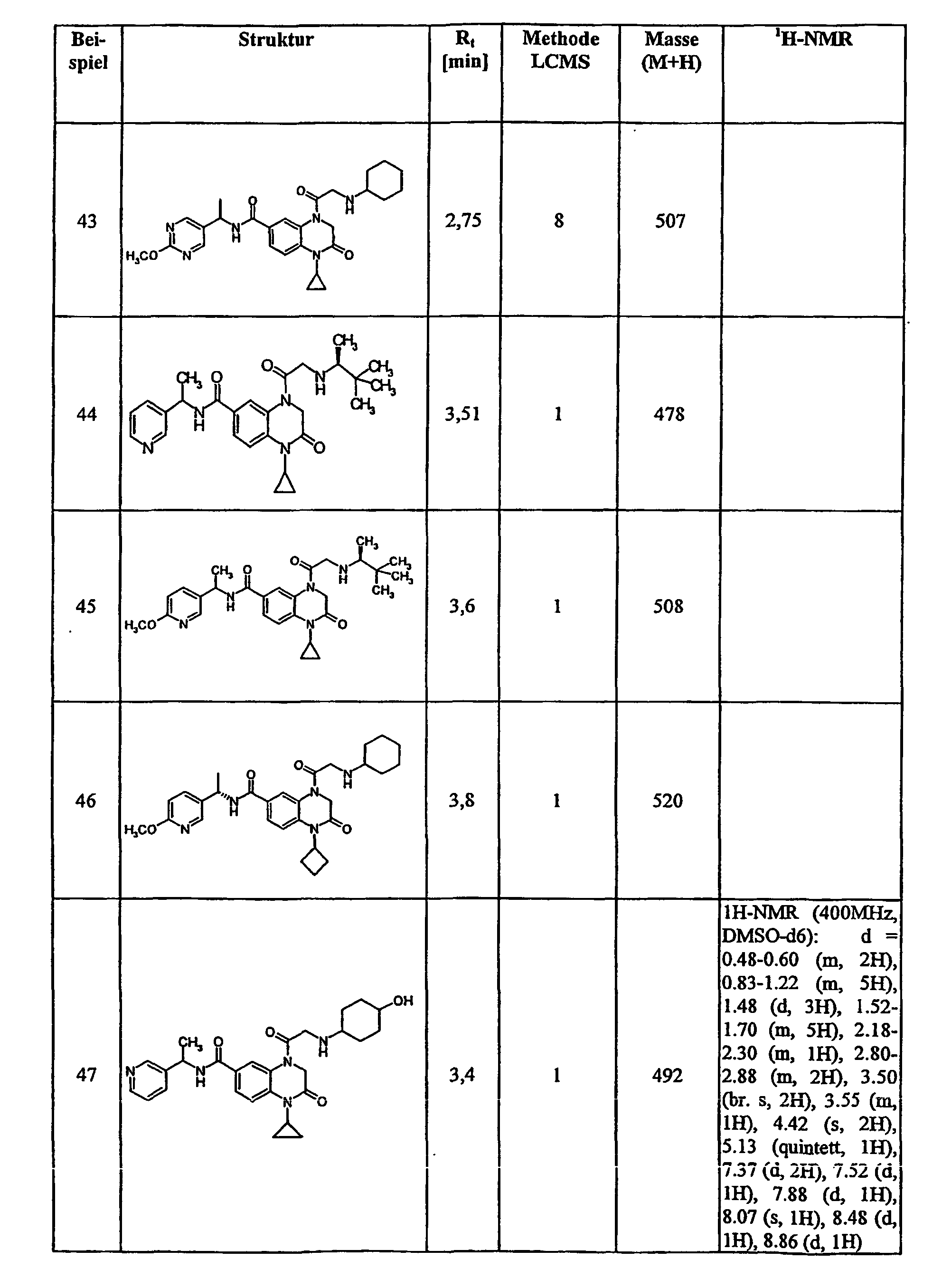

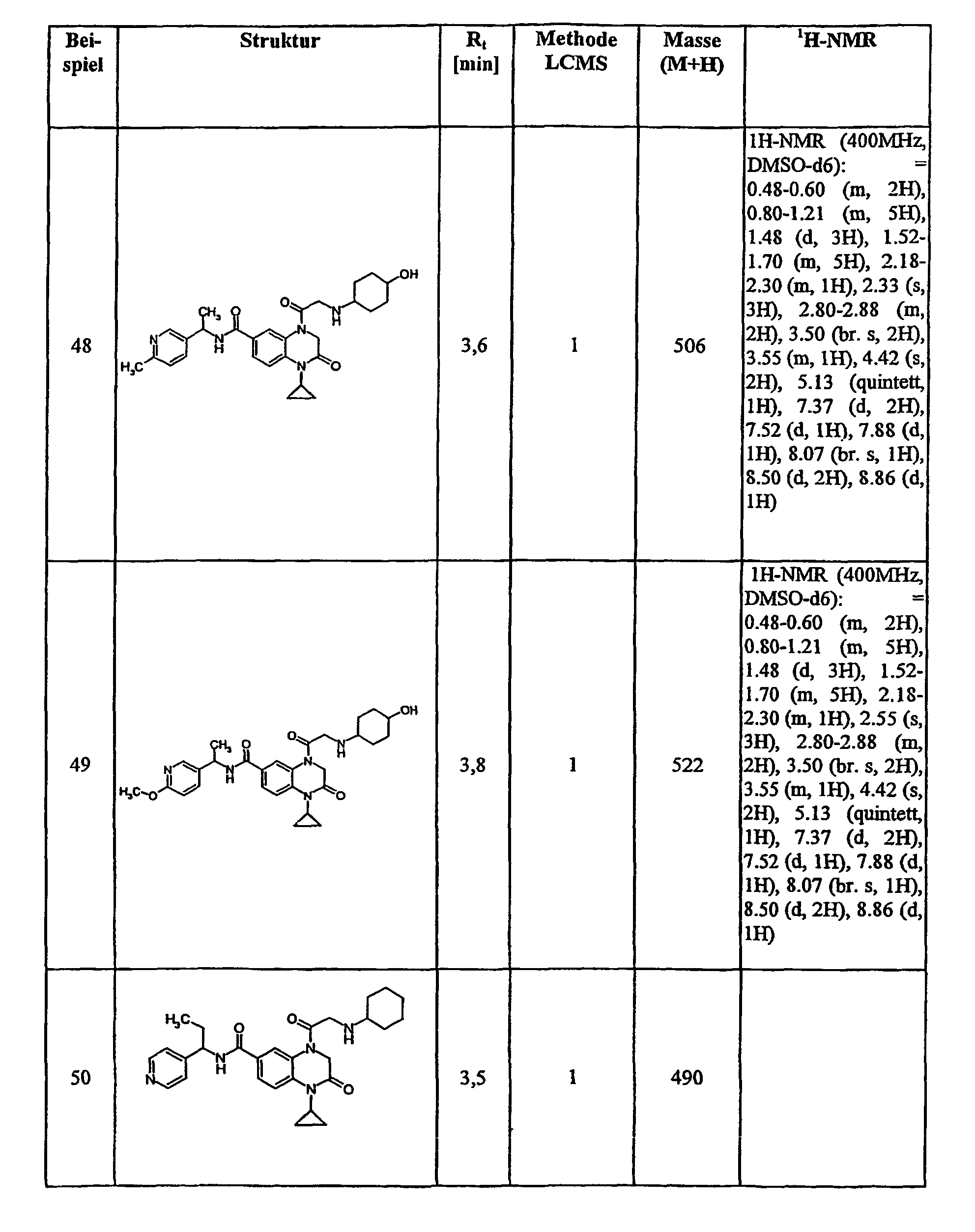

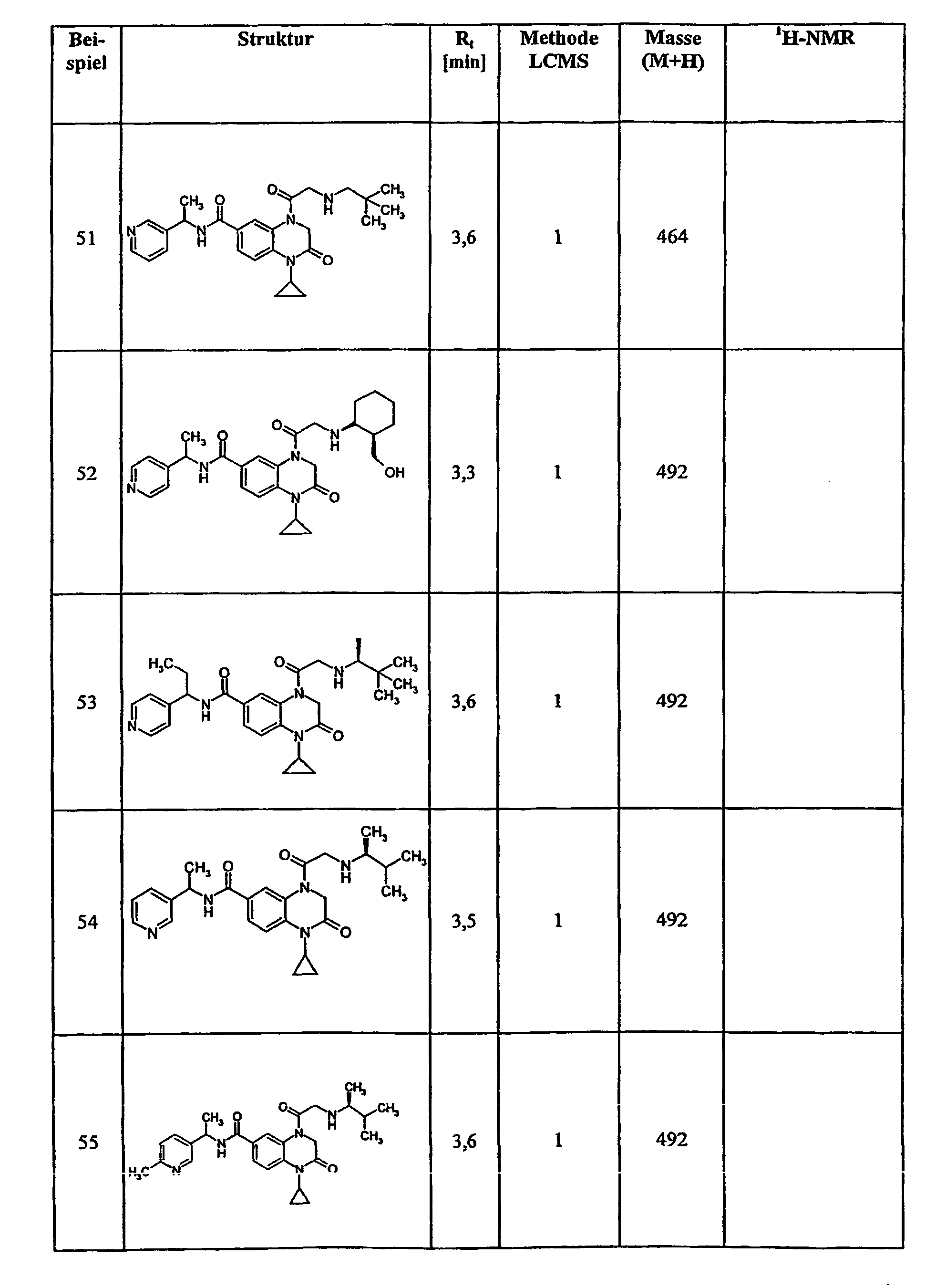

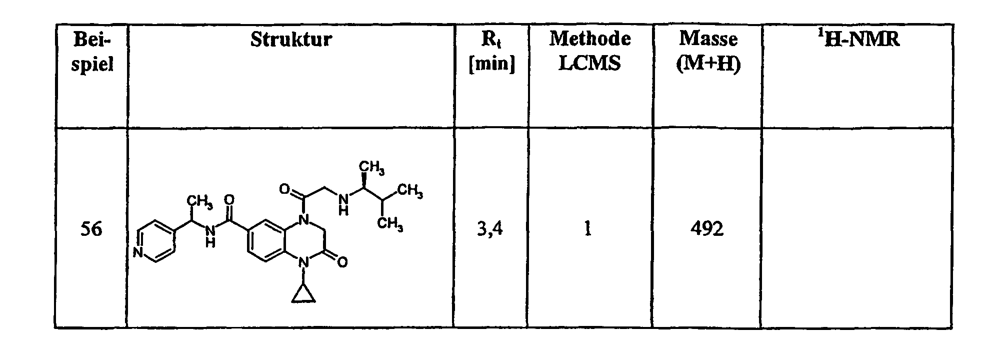

### B. Bewertung der physiologischen Wirksamkeit

### Abkürzungen:

- DMEM: Dulbecco's Modified Eagle Medium
- FCS: Fetal Calf Serum
- HEPES: 4-(2-hydroxyethyl)-1-piperazineethanesulfonic acid

### 1. in vitro-Tests zur Bestimmung der M2-Aktivität und -Selektivität

### a) Zellulärer, funktioneller in vitro-Test

Die Identifizierung von Agonisten des humanen M2-Acetylcholin-Rezeptors (M2AChR) sowie die Quantifizierung der Wirksamkeit der hier beschriebenen Substanzen erfolgte mit Hilfe einer rekombinanten Zelllinie. Die Zelle leitet sich ursprünglich von einer Ovarepithelzelle des Hamsters (Chinese Hamster Ovary, CHO K1, ATCC: American Type Culture Collection, Manassas, VA 20108, USA) ab. Die Testzelllinie expremiert konstitutiv eine modifizierte Form des calcium-sensitiven Photoproteins Aequorin, das nach Rekonstitution mit dem Co-Faktor Coelenterazin bei Erhöhungen der freien Calcium-Konzentration im inneren mitochondrialen Kompartiment Licht emittiert (Rizzuto R, Simpson AW, Brini M, Pozzan T.; *Nature* 358 (1992) 325-327). Zusätzlich ist die Zelle stabil transfiziert mit dem humanen M2AChR (Peralta EG, Ashkenazi A, Winslow JW, Smith DH, Ramachandran J, Capon, DJ, *EMBO Journal 6* (1987) 3923-3929) sowie dem Gen, das für das promiskuitive G_{α16}-Protein kodiert (Amatruda TT, Steele DA, Slepak VZ, Simon MI, *Proceedings in the National Academy of Science USA* 88 (1991), 5587-5591). Die resultierende M2AChR-Testzelle reagiert auf Stimulation des rekombinanten M2ACh-Rezeptors mit einer intrazellulären Freisetzung von Calcium-Ionen, die durch die resultierende Aequorin-Lumineszens mit einem geeignetem Luminometer quantifiziert werden kann (Milligan G, Marshall F, Rees S, *Trends in Pharmacological Sciences* 17 (1996) 235-237).

Zur Bestimmung der *in vitro*-Selektivität bezüglich der muskarinergen Acetylcholinrezeptorensubtypen M1 bis M5 werden entsprechende CHO K1 Zellen verwendet, die ebenfalls mit dem Gen des Calcium-sensitiven Photoproteins Aequorin und dem Gen des M1, M3 oder M5 Rezeptorsubtypen oder im Fall des M4 Rezeptorsubtypen zusätzlich mit dem Gen des promiskuitiven G_{α16}-Proteins stabil transfiziert sind.

Testablauf: Die Zellen werden am Tag vor dem Test in Kulturmedium (DMEM, 10% FCS, 2 mM Glutamine, 10 mM HEPES; Gibco Cat.# 21331-020; gehört jetzt zu: Invitrogen GmbH, 76131 Karlsruhe) in 384- (oder 1536-) Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (in mM: 140 NaCl, 5 KCl, 1 MgCl₂, 2 CaCl₂, 20 Glucose, 20 HEPES), das zusätzlich den Co-Faktor Coelenterazin (50 µM) enthält, ausgetauscht und die Mikrotiterplatte anschließend für weitere 3-4 Stunden inkubiert. Unmittelbar nach Übertragung der Testsubstanzen in die Löcher der Mikrotiterplatte wird das resultierende Lichtsignal im Luminometer gemessen. Die Ergebnisse sind in Tabelle A gezeigt:

**Tabelle A**

| **Beispiel Nr.** | **EC**_{**50**} **(nM)** |
|---|---|
| 2 | 1000 |
| 12 | 1800 |
| 7 | 5 |
| 11 | 320 |
| 5 | 37 |
| 22 | 150 |
| 23 | 18 |
| 24 | 32 |
| 25 | 6 |
| 26 | 16 |

### b) Bindungsstudien an humanen muskarinergen Acetylcholinrezeptoren

Stabil transfizierte CHO K1 Zellen, die den humanen muskarinergen M2 Rezeptor exprimieren, werden nach Erreichen von 80 % Konfluenz in 10 ml Bindungspuffer (20 mM 4-(2-Hydroxyethyl)-1-piperazinethansulfonsäure, 5 mM Magnesiumchlorid, pH 7,4) pro 175 cm² Zellkulturflasche suspendiert und mittels eines Ultra-Turrax-Gerätes homogenisiert. Die Homogenate werden 10 Minuten lang bei 1000 g und 4°C zentrifugiert. Der Überstand wird abgenommen und 30 min lang bei 20000 g und 4°C zentrifugiert. Das Membransediment mit den M2-Rezeptoren wird in 10 ml Bindungspuffer aufgenommen und bei -70°C gelagert.

Für den Bindungsversuch werden 2 nM ³H-Oxotremorin M (3200 GBq/mmol, PerkinElmer) 60 Minuten lang mit 100-1000 µg/ml M2-Membranen pro Ansatz (0,2ml) in Gegenwart der Testsubstanzen bei Raumtemperatur inkubiert. Die Inkubation wird durch eine 10 minütige Zentrifugation bei 10000 g und anschließendem Waschen mit 0.1% Rinderserumalbumin in Bindungspuffer bei 4°C abgestoppt. Es wird nochmals 10 Minuten lang bei 10000 x g und 4°C zentrifugiert.

Das Sediment wird in 0.1 ml 1 N Natronlauge resuspendiert und in Szintillationsröhrchen überführt. Nach Zugabe von 4 ml Ultima Gold Szintillator wird die an den Membranen gebundene Radioaktivität mittels eines LS6000 IC Szintillationszählers der Firma BeckmanCoulter quantifiziert. Die nicht-spezifische Bindung wird als Radioaktivität in Gegenwart von 10 µM Oxotremorin M definiert und beträgt in der Regel weniger als 5 % der gebundenen Gesamt-Radioaktivität. Die Bindungsdaten (IC₅₀ und Dissoziationskonstante Ki) werden mittels des Programmes GraphPad Prism Version 3.02 bestimmt.

### 2. in vivo-Test zum Nachweis der kardiovaskulären Wirkung

### a) Langendorff-Herz des Meerschweinchens

Narkotisierten Meerschweinchen wird nach Eröffnung des Brustkorbes das Herz entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

### b) Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden in Transcutol, Cremophor EL, H₂O (10%/20%/70%) in einem Volumen von 1 ml/kg oral verabreicht.

### c) Wirkung auf den mittleren Blutdruck von wachen, spontan hypertensiven Ratten

Kontinuierliche Blutdruckmessungen über 24 Stunden werden an spontan hypertonen 200-250g schweren sich frei bewegenden weiblichen Ratten (MOL:SPRD) durchgeführt. Dazu werden den Tieren chronisch Druckaufnehmer (Data Sciences Inc., St. Paul, MN, USA) in die absteigende Bauchaorta unterhalb der Nierenarterie implantiert und der damit verbundene Sender in der Bauchhöhle fixiert. Die Tiere werden einzeln in Typ III Käfigen, die auf den individuellen Empfängerstationen positioniert sind, gehalten und werden an einem 12-Stunden Hell/Dunkel-Rhythmus angepasst. Wasser und Futter stehen frei zur Verfügung. Zur Datenerfassung wird der Blutdruck jeder Ratte alle 5 Minuten für 10 Sekunden registriert. Die Messpunkte werden jeweils für eine Periode von 15 Minuten zusammengefasst und der Mittelwert aus diesen Werten berechnet. Die Prüfverbindungen werden in einer Mischung aus Transcutol (10%), Cremophor (20%), H₂O (70%) gelöst und mittels Schlundsonde in einem Volumen von 2 ml/kg Körpergewicht oral verabreicht. Die Prüfdosen liegen zwischen 0,3 -30 mg/kg Körpergewicht.

### d) Blutdruck- und Herzfrequenzmessungen an narkotisierten Hunden

Die Experimente werden in Hunden (Mongrel) beiderlei Geschlechts mit einem Körpergewicht zwischen 20 und 30 kg durchgeführt. Die Narkose wird durch eine langsame i.v. Injektion von 25 mg/kg Thiopental (Trapanal®) eingeleitet und während des Experiments durch eine kontinuierliche Infusion von 0.08 mg/kg/h Fentanyl (Fentanyl®) und 0.25 mg/kg/h Droperidol (Dehydrobenzperidol®) fortgeführt. Alloferin (0.02 mg/kg/h) wird als Muskelrelaxans hünugefügt. Die Hunde werden künstlich mit 1 Teil Lachgas und 3 Teilen Sauerstoff beatmet. Die Prüfsubstanzen werden intravenös über die Femoralvene appliziert.

Ein MillarTip-catheter zur Aufnahme des linksventrikulären Drucks bzw. Berechnung der Kontraktilität wird über die A. carotis in den linken Ventrikel geführt. Ein Hohlkatheter wird über die A. femoralis in die Aorta eingeführt und zur Messung des peripheren Blutdrucks mit einem Druckaufnehmer verbunden. Nach einer linksseitigen Thorakotomie wird der Ramus circumflexus (LCX) oder der Ramus interventricularis (LAD) der linken Koronararterie freipräpariert und ein elektromagnetischer Flußkopf zur Messung des Koronarflusses angelegt. Das EKG wird über eine Extremitätenableitung und einen EKG-Verstärker aufgenommen, die Herzfrequenz und EKG-Parameter werden über das gemessene EKG ermittelt. Die Sauerstoffsättigung am Koronarsinus wird über einen Swan-Gantz Oximetrie TD Katheter bestimmt.

### B. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Pesskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.
Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

## Patentansprüche

1. Verbindungen der Formel 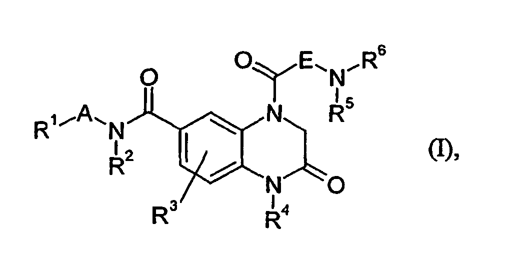 in welcher
A für eine C₁-C₆-Alkandiylkette steht, die gegebenenfalls mit einer oder zwei Hydroxygruppen substituiert ist,
E für eine C₁-C₆-Alkandiylkette steht,
R¹ für Heteroaryl steht, wobei Heteroaryl gegebenenfalls substituiert ist mit 1 bis 3 Substituenten, unabhängig voneinander ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl,
R² für Wasserstoff, Alkyl oder Cycloalkyl steht,
R³ für Wasserstoff, Halogen, Alkyl oder Alkoxy steht,
R⁴ für Alkyl oder Cycloalkyl steht, wobei Alkyl und Cycloalkyl gegebenenfalls substituiert sind mit 1 bis 3 Substituenten, unabhängig voneinander ausgewählt aus der Gruppe bestehend aus Halogen, Hydroxy, Amino, Alkyl, Alkoxy, Alkylamino, Alkoxycarbonyl, Aminocarbonyl und Alkylamino-carbonyl,
R⁵ für Wasserstoff, Alkyl oder Cycloalkyl steht,
und
R⁶ für Alkyl, Cycloalkyl, Heterocyclyl, Aryl oder Heteroaryl steht, wobei Alkyl, Cycloalkyl, Heterocyclyl, Aryl oder Heteroaryl gegebenenfalls substituiert sind mit 1 bis 3 Substituenten unabhängig voneinander ausgewählt aus der Gruppe bestehend aus Halogen, Hydroxy, Amino, Trifluormethyl, Nitro, Cyano, Alkyl, das seinerseits mit einer oder zwei Hydroxygruppen substituiert sein kann, Alkoxy, Alkylamino, Alkoxycarbonyl, Aminocarbonyl und Alkylaminocarbonyl,
oder
R⁵ und R⁶ bilden zusammen mit dem Stickstoffatom an das sie gebunden sind einen stickstoffhaltigen nicht-aromatischen Heterocyclyl-Ring, wobei Heterocyclyl gegebenenfalls substituiert ist mit 1 bis 3 Substituenten unabhängig voneinander ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl
und ihre Salze, Solvate und Solvate der Salze.

2. Verbindungen der Formel (I) nach Anspruch 1,
in welcher
A für eine C₁-C₆-Alkandiylkette steht,
E für eine C₁-C₆-Alkandiylkette steht,
R¹ für Heteroaryl steht, wobei Heteroaryl gegebenenfalls substituiert ist mit 1 bis 3 Substituenten, unabhängig voneinander ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl,
R² für Wasserstoff steht,
R³ für Wasserstoff steht,
R⁴ für Alkyl oder Cycloalkyl steht, wobei Alkyl und Cycloalkyl gegebenenfalls substituiert sind mit 1 bis 3 Substituenten, unabhängig voneinander ausgewählt aus der Gruppe bestehend aus Alkyl, Alkoxy und Alkylamino,
R⁵ für Wasserstoff steht,
und
R⁶ für Alkyl, Cycloalkyl, Heterocyclyl, Aryl oder Heteroaryl steht, wobei Alkyl, Cycloalkyl, Heterocyclyl, Aryl oder Heteroaryl gegebenenfalls substituiert sind mit 1 bis 3 Substituenten unabhängig voneinander, ausgewählt aus der Gruppe bestehend aus Halogen, Hydroxy, Amino, Trifluormethyl, Nitro, Cyano, Alkyl, Alkoxy, Alkylamino, Alkoxycarbonyl, Aminocarbonyl und Alkylaminocarbonyl
und ihre Salze, Solvate und Solvate der Salze.

3. Verbindungen der Formel (I) nach Anspruch 1,
in welcher
A für Methylen oder Ethan-1,1-diyl steht,
E für Methylen steht,
R¹ für 5- oder 6-gliedriges Heteroaryl steht, wobei Heteroaryl gegebenenfalls substituiert ist mit 1 bis 3 Substituenten, unabhängig voneinander ausgewählt aus der Gruppe bestehend aus Halogen, Hydroxy, Amino, Alkyl, Alkoxy, Alkylamino, Alkoxycarbonyl, Aminocarbonyl und Alkylaminocarbonyl,
R² für Wasserstoff steht,
R³ für Wasserstoff steht,
R⁴ für Cycloalkyl steht, wobei Cycloalkyl gegebenenfalls substituiert ist mit 1 oder 2 Alkylsubstituenten,
R⁵ für Wasserstoff steht,
und
R⁶ für Alkyl oder Cycloalkyl steht, wobei Cycloalkyl gegebenenfalls substituiert ist mit 1 bis 3 Substituenten, unabhängig voneinander ausgewählt aus der Gruppe bestehend aus Alkyl, Alkoxy, Alkylamino, Alkoxycarbonyl, Aminocarbonyl und Alkylaminocarbonyl
und ihre Salze, Solvate und Solvate der Salze.

4. Verbindungen der Formel (I) nach Anspruch 1,
in welcher
A für Ethan-1,1-diyl steht,
E für Methylen steht,
R¹ für 6-gliedriges Heteroaryl steht, wobei Heteroaryl gegebenenfalls substituiert ist mit 1 oder 2 Substituenten, unabhängig voneinander ausgewählt aus der Gruppe bestehend aus Methyl, Ethyl, Methoxy und Ethoxy,
R² für Wasserstoff steht,
R³ für Wasserstoff steht,
R⁴ für Cyclopropyl steht,
R⁵ für Wasserstoff steht,
und
R⁶ für Cyclohexyl oder Cyclopentyl steht, wobei Cyclohexyl oder Cyclopentyl gegebenenfalls substituiert sind mit 1 oder 2 Substituenten, unabhängig voneinander ausgewählt aus der Gruppe bestehend aus Methyl, Ethyl, Methoxy und Ethoxy
und ihre Salze, Solvate und Solvate der Salze.

5. Verfahren zur Herstellung der Verbindungen der Formel (I), wie in Anspruch 1 definiert, **dadurch gekennzeichnet, dass** man Verbindungen der Formel 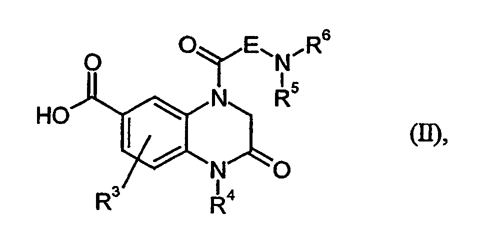 in welcher
E, R³, R⁴, R⁵ und R⁶ die in Anspruch 1 angegebene Bedeutung aufweisen,
mit Verbindungen der Formel 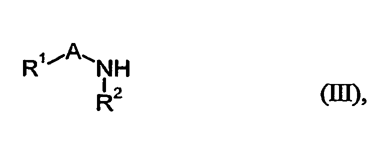 in welcher
A, R¹ und R² die in Anspruch 1 angegebene Bedeutung aufweisen,
umsetzt.

6. Verbindungen der Formel (I), wie in Anspruch 1 definiert, zur Bekämpfung von Erkrankungen.

7. Arzneimittel, enthaltend mindestens eine Verbindung der Formel (I), wie in Anspruch 1 definiert, und mindestens einen weiteren Hilfsstoff.

8. Arzneimittel, enthaltend mindestens eine Verbindung der Formel (I), wie in Anspruch 1 definiert, und mindestens einen weiteren Wirkstoff.

9. Verwendung von Verbindungen nach einem der Ansprüche 1 bis 3 zur Herstellung eines Arzneimittels zur Behandlung und/oder Prophylaxe von kardiovaskulären Erkrankungen.

## Claims

1. Compounds of the formula 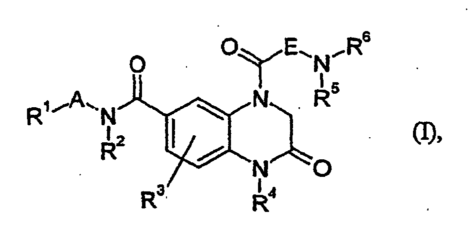 in which
A is a C₁-C₆-alkanediyl chain which is optionally substituted by one or two hydroxy groups,
E is a C₁-C₆-alkanediyl chain,
R¹ is heteroaryl, where heteroaryl is optionally substituted by 1 to 3 substituents independently of one another selected from the group consisting of halogen, hydroxy, amino, trifluoromethyl, nitro, cyano, alkyl, alkoxy, alkylamino, alkoxycarbonyl, aminocarbonyl and alkylaminocarbonyl,
R² is hydrogen, alkyl or cycloalkyl,
R³ is hydrogen, halogen, alkyl or alkoxy,
R⁴ is alkyl or cycloalkyl, where alkyl and cycloalkyl are optionally substituted by 1 to 3 substituents independently of one another selected from the group consisting of halogen, hydroxy, amino, alkyl, alkoxy, alkylamino, alkoxycarbonyl, aminocarbonyl and alkylaminocarbonyl,
R⁵ is hydrogen, alkyl or cycloalkyl,
and
R⁶ is alkyl, cycloalkyl, heterocyclyl, aryl or heteroaryl, where alkyl, cycloalkyl, heterocyclyl, aryl or heteroaryl are optionally substituted by 1 to 3 substituents independently of one another selected from the group consisting of halogen, hydroxy, amino, trifluoromethyl, nitro, cyano, alkyl which may in turn be substituted by one or two hydroxy groups, or alkoxy, alkylamino, alkoxycarbonyl, aminocarbonyl and alkylaminocarbonyl,
or
R⁵ and R⁶ form together with the nitrogen atom to which they are bonded a nitrogen-containing nonaromataic ring, where heterocyclyl is optionally substituted by 1 to 3 substituents independently of one another selected from the group consisting of halogen, hydroxy, amino, trifluoromethyl, nitro, cyano, alkyl, alkoxy, alkylamino, alkoxycarbonyl, aminocarbonyl and alkylaininocarbonyl,
and the salts, solvates and solvates of the salts thereof.

2. Compounds of the formula (I) according to Claim 1,
in which
A is a C₁-C₆-alkanediyl chain,
E is a C₁-C₆-alkanediyl chain,
R¹ is heteroaryl, where heteroaryl is optionally substituted by 1 to 3 substituents independently of one another selected from the group consisting of halogen, hydroxy, amino, trifluoromethyl, nitro, cyano, alkyl, alkoxy, alkylamino, alkoxycarbonyl, aminocarbonyl and alkylaminocarbonyl,
R² is hydrogen,
R³ is hydrogen,
R⁴ is alkyl or cycloalkyl, where alkyl and cycloalkyl are optionally substituted by 1 to 3 substituents independently of one another selected from the group consisting of alkyl, alkoxy and alkylamino,
R⁵ is hydrogen,
and
R⁶ is alkyl, cycloalkyl, heterocyclyl, aryl or heteroaryl, where alkyl, cycloalkyl, heterocyclyl, aryl or heteroaryl are optionally substituted by 1 to 3 substituents independently of one another selected from the group consisting of halogen, hydroxy, amino, trifluoromethyl, nitro, cyano, alkyl, alkoxy, alkylamino, alkoxycarbonyl, aminocarbonyl and alkylaminocarbonyl,
and the salts, solvates and solvates of the salts thereof.

3. Compounds of the formula (I) according to Claim 1,
in which
A is methylene or ethane-1,1-diyl,
E is methylene,
R¹ is 5- or 6-membered heteroaryl, where heteroaryl is optionally substituted by 1 to 3 substituents independently of one another selected from the group consisting of halogen, hydroxy, amino, alkyl, alkoxy, alkylamino, alkoxycarbonyl, aminocarbonyl and alkylaminocarbonyl,
R² is hydrogen,
R³ is hydrogen,
R⁴ is cycloalkyl, where cycloalkyl is optionally substituted by 1 or 2 alkyl substituents,
R⁵ is hydrogen,
and
R⁶ is alkyl or cycloalkyl, where cycloalkyl is optionally substituted by 1 to 3 substituents independently of one another selected from the group consisting of alkyl, alkoxy, alkylamino, alkoxycarbonyl, aminocarbonyl and alkylaminocarbonyl,
and the salts, solvates and solvates of the salts thereof.

4. Compounds of the formula (I) according to Claim 1,
in which
A is ethane-1,1-diyl,
E is methylene,
R¹ is 6-membered heteroaryl, where heteroaryl is optionally substituted by 1 or 2 substituents independently of one another selected from the group consisting of methyl, ethyl, methoxy and ethoxy,
R² is hydrogen,
R³ is hydrogen,
R⁴ is cyclopropyl,
R⁵ is hydrogen,
and
R⁶ is cyclohexyl or cyclopentyl, where cyclohexyl or cyclopentyl are optionally substituted by 1 or 2 substituents independently of one another selected from the group consisting of methyl, ethyl, methoxy and ethoxy,
and the salts, solvates and solvates of the salts thereof.

5. Process for preparing the compounds of the formula (I) as defined in Claim 1, **characterized in that** the compounds of the formula 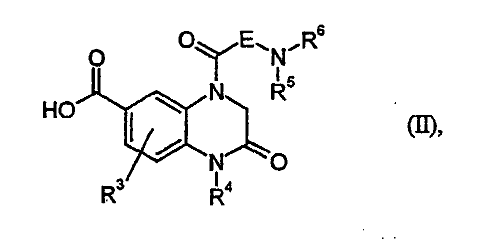 in which
E, R³, R⁴, R⁵ and R⁶ have the meaning stated in Claim 1, are reacted with compounds of the formula 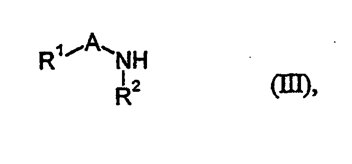 in which
A, R¹ and R² have the meaning stated in Claim 1.

6. Compounds of the formula (I) as defined in Claim 1 for controlling diseases.

7. Medicament comprising at least one compound of the formula (I) as claimed in Claim 1, and at least one further excipient.

8. Medicament comprising at least one compound of the formula (I) as defined in Claim 1, and at least one further active ingredient.

9. Use of compounds according to any of Claims 1 to 3 for producing a medicament for the treatment and/or prophylaxis of cardiovascular disorders.

## Revendications

1. Composés de formule 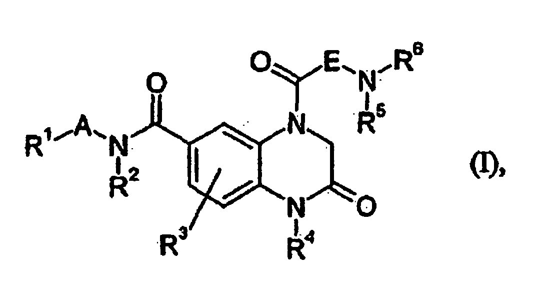 dans laquelle
A représente une chaîne alcanediyle en C₁ à C₆, qui est éventuellement substituée avec un ou deux groupes hydroxy,
E représente une chaîne alcanediyle en C₁ à C₆,
R¹ représente un reste hétéroaryle qui est éventuellement substitué avec 1 à 3 substituants choisis, indépendamment les uns des autres, dans le groupe constitué d'halogène, hydroxy, amino, trifluorométhyle, nitro, cyano, alkyle, alkoxy, alkylamino, alkoxycarbonyle, aminocarbonyle et alkylaminocarbonyle,
R² représente l'hydrogène, un reste alkyle ou cycloalkyle,
R³ représente l'hydrogène, un halogène, un reste alkyle ou alkoxy,
R⁴ est un reste alkyle ou cycloalkyle, chacun éventuellement substitué avec 1 à 3 substituants choisis, indépendamment les uns des autres, dans le groupe constitué d'halogène, hydroxy, amino, alkyle, alkoxy, alkylamino, alkoxycarbonyle, aminocarbonyle et alkylaminocarbonyle,
R⁵ représente l'hydrogène, un reste alkyle ou cycloalkyle,
et
R⁶ représente un reste alkyle, cycloalkyle, hétérocyclyle, aryle ou hétéroaryle, chacun éventuellement substitué avec 1 à 3 substituants choisis indépendamment les uns des autres dans le groupe constitué d'halogène, hydroxy, amino, trifluorométhyle, nitro, cyano, alkyle qui peut lui-même être substitué avec un ou deux groupes hydroxy, alkoxy, alkylamino, alkoxycarbonyle, aminocarbonyle et alkylaminocarbonyle,
ou bien
R⁵ et R⁶ forment conjointement avec l'atome d'azote auquel ils sont liés un noyau hétérocyclyle non aromatique azoté, qui porte éventuellement 1 à 3 substituants choisis, indépendamment les uns des autres, dans le groupe constitué d'halogène, hydroxy, amino, trifluorométhyle, nitro, cyano, alkyle, alkoxy, alkylamino, alkoxycarbonyle, aminocarbonyle et alkylaminocarbonyle,
et leurs sels, leurs produits de solvatation et les produits de solvatation des sels.

2. Composés de formule (I) suivant la revendication 1,
formule dans laquelle
A représente une chaîne alcanediyle en C₁ à C₆,
E représente une chaîne alcanediyle en C₁ à C₆,
R¹ est un reste hétéroaryle qui porte éventuellement 1 à 3 substituants choisis, indépendamment les uns des autres, dans le groupe constitué d'halogène, hydroxy, amino, trifluorométhyle, nitro, cyano, alkyle, alkoxy, alkylamino, alkoxycarbonyle, aminocarbonyle et alkylaminocarbonyle,
R² représente l'hydrogène,
R³ représente l'hydrogène,
R⁴ est un reste alkyle ou cycloalkyle portant chacun le cas échéant 1 à 3 substituants choisis, indépendamment les uns des autres, dans le groupe constitué d'alkyle, alkoxy et alkylamino,
R⁵ représente l'hydrogène,
et
R⁶ est un reste alkyle, cycloalkyle, hétérocyclyle, aryle ou hétéroaryle, portant chacun le cas échéant 1 à 3 substituants, choisis indépendamment les uns des autres, dans le groupe constitué d'halogène, hydroxy, amino, trifluorométhyle, nitro, cyano, alkyle, alkoxy, alkylamino, alkoxycarbonyle, aminocarbonyle et alkylaminocarbonyle,
et leurs sels, leurs produits de solvatation et les produits de solvatation des sels.

3. Composés de formule (I) suivant la revendication 1,
formule dans laquelle
A est un reste méthylène ou éthane-1,1-diyle,
E est un reste méthylène,
R¹ est un reste hétéroaryle pentagonal ou hexagonal, qui porte éventuellement 1 à 3 substituants choisis, indépendamment les uns des autres, dans le groupe constitué d'halogène, hydroxy, amino, alkyle, alkoxy, alkylamino, alkoxycarbonyle, aminocarbonyle et alkylaminocarbonyle,
R² représente l'hydrogène,
R³ représente l'hydrogène,
R⁴ est un reste cycloalkyle portant éventuellement 1 ou 2 substituants alkyle,
R⁵ représente l'hydrogène,
et
R⁶ est un reste alkyle ou cycloalkyle, le reste cycloalkyle portant éventuellement 1 à 3 substituants choisis, indépendamment les uns des autres, dans le groupe constitué d'alkyle, alkoxy, alkylamino, alkoxycarbonyle, aminocarbonyle et alkylaminocarbonyle,
et leurs sels, leurs produits de solvatation et les produits de solvatation des sels.

4. Composés de formule (I) suivant la revendication 1, formule dans laquelle
A est un reste éthane-1,1-diyle,
E est un reste méthylène,
R¹ est un reste hétéroaryle hexagonal portant éventuellement 1 ou 2 substituants choisis indépendamment l'un de l'autre dans le groupe constitué de méthyle, éthyle, méthoxy et éthoxy,
R² représente l'hydrogène,
R³ représente l'hydrogène,
R⁴ est un reste cyclopropyle,
R⁵ représente l'hydrogène,
et
R⁶ est un reste cyclohexyle ou cyclopentyle portant chacun le cas échéant 1 ou 2 substituants choisis indépendamment l'un de l'autre dans le groupe constitué de méthyle, éthyle, méthoxy et éthoxy,
et leurs sels, leurs produits de solvatation et les produits de solvatation des sels.

5. Procédé de production des composés de formule (I) tels que définis dans la revendication 1, **caractérisé en ce qu'**on fait réagir des composés de formule 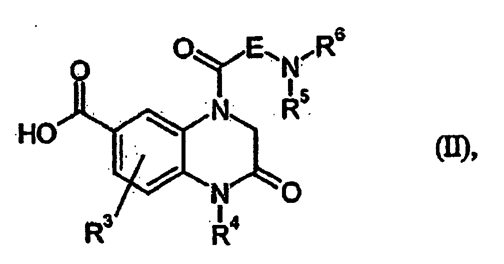 dans laquelle
E, R³, R⁴, R⁵ et R⁶ ont la définition indiquée dans la revendication 1,
avec des composés de formule 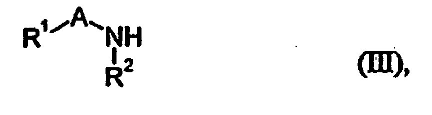 dans laquelle
A, R¹ et R² ont la définition indiquée dans la revendication 1.

6. Composés de formule (I) tels que définis dans la revendication 1, destinés à combattre des maladies.

7. Médicament contenant au moins un composé de formule (I) tel que défini dans la revendication 1 et au moins une autre substance auxiliaire.

8. Médicament contenant au moins un composé de formule (I) tel que défini dans la revendication 1 et au moins une autre substance active.

9. Utilisation de composés suivant l'une des revendications 1 à 3 pour la préparation d'un médicament destiné au traitement et/ou à la prophylaxie de maladies cardio-vasculaires.
